(19) Europäisches Patentamt
European Patent Office
Office européen des brevets

(11) **EP 2 274 634 B1**

(12) **EUROPÄISCHE PATENTSCHRIFT**

(45) Veröffentlichungstag und Bekanntmachung des Hinweises auf die Patenterteilung:
**20.03.2013 Patentblatt 2013/12**

(21) Anmeldenummer: **09734645.6**

(22) Anmeldetag: **21.04.2009**

(51) Int Cl.:
*G01R 33/38* (2006.01)    *G01R 33/44* (2006.01)
*H05H 7/02* (2006.01)    *H05H 13/04* (2006.01)

(86) Internationale Anmeldenummer:
**PCT/EP2009/002902**

(87) Internationale Veröffentlichungsnummer:
**WO 2009/130002 (29.10.2009 Gazette 2009/44)**

(54) **MAGNETFELDERZEUGUNGSVORRICHTUNG**

MAGNETIC FIELD GENERATING MEANS

DISPOSITIF DE PRODUCTION DE CHAMP MAGNÉTIQUE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **18.11.2008 DE 102008057815**
**23.04.2008 DE 102008020445**

(43) Veröffentlichungstag der Anmeldung:
**19.01.2011 Patentblatt 2011/03**

(73) Patentinhaber: **GSI Helmholtzzentrum für Schwerionenforschung GmbH**
**64291 Darmstadt (DE)**

(72) Erfinder: **STEINER, Rudolf**
**65366 Gelsenheim (DE)**

(74) Vertreter: **Rück, Dorothee Maria**
**GSI**
**Helmholtzzentrum für Schwerionenforschung GmbH**
**Planckstraße 1**
**D-64291 Darmstadt (DE)**

(56) Entgegenhaltungen:
**EP-A1- 0 899 576    EP-A1- 1 603 142**
**DE-A1- 4 419 061    DE-A1- 19 702 831**
**DE-T2- 69 831 538    US-B2- 6 635 883**

EP 2 274 634 B1

**Beschreibung**

Die Erfindung betrifft eine Magnetfelderzeugungsvorrichtung mit wenigstens einem Magnetgap zur Aufnahme von Gegenständen, auf die ein Magnetfeld, das von wenigstens einem Magnetfelderzeugungsmittel der Magnetfelderzeugungsvorrichtung erzeugt wird, einwirkt. Die Erfindung betrifft weiterhin eine Anlage mit einer derartigen Magnetfelderzeugungsvorrichtung. Darüber hinaus betrifft die Erfindung auch ein Verfahren zum Ansteuern einer derartigen Magnetfelderzeugungsvorrichtung bzw. ein Verfahren zum Ansteuern einer Anlage mit einer derartigen Magnetfelderzeugungsvorrichtung.

Geräte und Einrichtungen, deren Betrieb oder Funktion zu einem erheblichen Anteil auf der Erzeugung starker, ggf. auch sich verändernder Magnetfelder beruht, finden sich in zunehmendem Maße bei Geräten, die zu Fertigungszwecken, Wartungszwecken oder auch im medizinischen Bereich verwendet werden. Dies gilt auch für Geräte, die noch vor wenigen Jahren ausschließlich zu Zwecken der Grundlagenforschung verwendet wurden.

Hier ist beispielsweise an Teilchenbeschleuniger zu therapeutischen Zwecken zu denken. Teilchenbeschleuniger können insbesondere zu Zwecken der Tumortherapie verwendet werden. Insbesondere können auch inoperable Tumore, speziell Gehirntumore, mit Hilfe von Teilchenbeschleunigern erfolgreich therapiert werden. Als Teilchen, die in solchen therapeutischen Teilchenbeschleunigern beschleunigt werden, werden nicht nur Elektronen, sondern in jüngster Zeit auch Ionen (meist Kohlenstoffionen) verwendet. Als Teilchenbeschleuniger finden nicht nur Linearbeschleuniger, sondern auch Synchrotrone (Beschleunigerringe) Verwendung.

Weitere Beispiele für Geräte, deren Betrieb oder Funktion zu einem erheblichen Anteil auf der Erzeugung starker, sich ggf. verändernder Magnetfelder beruht, sind Geräte im Fertigungsbereich, wie beispielsweise Geräte für das Induktionshärten. Auch im medizinischen Bereich gibt es weitere Einsatzgebiete, wie beispielsweise bildgebende NMR-Diagnosegeräte (NMR für "nuclear magnetic resonance").

Solche Geräte sind in der DE 4419061, der EP 1603142 und der US 6635883 offenbart.

Während bei reinen Forschungsanwendungen die Wirtschaftlichkeit der Anlage eine eher geringe Priorität aufweist, erfordern Anwendungen im Fertigungsbereich, im Wartungsbereich und im medizinischen Bereich in zunehmendem Maße einen möglichst wirtschaftlichen Betrieb der Anlagen.

Ein Problem bei heute verwendeten Magneten liegt in der Reproduzierbarkeit der Magnetfelder. Dies ist insbesondere bei Magneten der Fall, die ein zeitlich veränderliches Magnetfeld erzeugen sollen. Speziell bei sich häufig und relativ schnell zeitlich verändernden Magnetfeldern tritt darüber hinaus das Problem auf, dass ein bestimmtes Soll-Magnetfeld möglichst schnell erreicht werden soll.

Um beispielsweise bei einem Synchrotron einen möglichst wirtschaftlichen Betrieb zu erzielen, ist es erwünscht, dass im zeitlichen Mittel ein möglichst hoher Anteil an Teilchenstrahlaustragszeit erzielt wird. Dies kann man dadurch erreichen, dass man die Zeitanteile, in denen sich das Synchrotron in den restlichen Betriebsmodi befindet, so weit wie möglich verringert. Dazu werden beispielsweise die Zeiten, die für die Injektion und die Beschleunigung des Teilchenstrahls im Synchrotron verwendet werden, möglichst kurz gehalten. Dies wiederum erfordert es, den Zeitbedarf für den Aufbau (das Hochfahren), den Abbau (das Herunterfahren) und die Stabilisierung der Magnetfelder, die für die Strahlumlenkung (Dipolmagnete) und für die Strahlfokussierung (Quadrupolmagnete) verwendet werden, möglichst klein zu halten.

Bei Teilchenbeschleunigern erfolgt die Ansteuerung der Magnete derzeit in aller Regel durch Stromquellen, die die Magnete mit einer vorgegebenen (in der Regel zeitlich veränderlichen) Stromstärke beaufschlagen. Dazu haben sich sogenannte DCCT (für "direct current to current transformer") über die Jahre bewährt. Um die Beziehung zwischen dem durch die Magnetspulen fließenden Strom und dem Magnetfeld zu ermitteln, werden die Magnete vorab in einem Labor vermessen. Für jede angelegte Stromstärke wird das im Magneten herrschende Magnetfeld gemessen. Dazu wird eine entsprechende Sonde - in der Praxis meist ein NMR oder eine Hallsonde - verwendet. Mit den so gewonnenen Messdaten wird ein Kennlinienfeld erstellt, das anschließend zur Ansteuerung des Magneten verwendet wird. Da sich das Magnetfeld auch innerhalb des Magneten ortsabhängig verändert, sind zur Ermittlung der Ortsabhängigkeit zusätzliche Labormessungen erforderlich.

Die Erstellung von Kennlinienfeldern im Rahmen von Labormessungen wird durch die Remanenz von magnetischen Materialien erschwert. Bei den derzeit in Beschleunigeranlagen verwendeten Magneten (Dipolmagnete und Quadrupolmagnete) handelt es sich fast ausnahmslos um Elektromagnete mit normal leitenden Stromspulen mit Kernen und Jochen aus weichmagnetischem Material (meist miteinander verklebte Eisenbleche). Wenn der Elektromagnet durch das Anlegen von elektrischem Strom ein Magnetfeld erzeugt, wird zwangsläufig das weichmagnetische Material magnetisiert. Auch wenn der elektrische Strom anschließend abgeschaltet wird, verbleibt eine Restmagnetisierung des weichmagnetischen Materials, die sogenannte Remanenz. Dadurch verbleibt auch im Magnetgap des Elektromagneten ein Magnetfeld. Die Remanenzfeldstärke ist eine statische Eigenschaft und klingt nicht mit der Zeit ab. Die Stärke der Remanenzfeldstärke hängt dabei nicht nur von den Materialien und der Bauart des Elektromagneten ab, sondern auch von der Magnetisierungsvorgeschichte des Elektromagneten bzw. des weichmagnetischen Materials. Dadurch kann man einem bestimmten, konstanten Magnetstromwert $I_n$ nicht notwendigerweise

eine stets gleiche Magnetfeldstärke zuordnen.

Um überhaupt von einem bestimmten Magnetstromwert $I_n$ auf die vom Elektromagneten erzeugte Magnetfeldstärke schließen zu können, ist es erforderlich, eine festgelegte Prozedur zum Einfahren des Magnetstromwerts $I_n$ einzuhalten. Dadurch wird eine definierte Magnetisierungsvorgeschichte erreicht. Dies wird als "konditionieren" bezeichnet. Das übliche Konditionierungsverfahren besteht darin, den Magneten zu seinem Maximalwert zu steuern (und damit die weichmagnetischen Materialien in die Sättigung zu treiben), ihn anschließend auf Nullstrom zurückzufahren und von dort aus den Einstellwert anzufahren. Die Reproduzierbarkeit des Magnetfeldes ist nach dem Konditionieren üblicherweise besser als $10^{-4}$ T (bei Maximalfeldstärken von üblicherweise 1,5 T bis 2 T). Das maximale Remanenzfeld B, liegt bei Strahlführungsmagneten typischerweise zwischen $\pm 1 \cdot 10^{-3}$ T und $\pm 3 \cdot 10^{-3}$ T. Bei unipolar betriebenen Magneten ist das maximale Remanenzfeld B, typischerweise kleiner und liegt im Bereich von etwa $\pm 2 \cdot 10^{-4}$ T bis $\pm 4 \cdot 10^{-4}$ T. Die im Labor ermittelten Kennlinienfelder basieren auf einer derartigen definierten Konditionierung.

Im Gebiet von Teilchenbeschleunigern bedeutet die Remanenz, dass der vorangegangene Beschleunigungszyklus bzw. die vorangegangenen Beschleunigungszyklen grundsätzlich einen Einfluss auf das Verhalten des/der Magneten beim anschließenden Beschleunigungszyklus haben. Da bei Teilchenbeschleunigern Magnetfeldvariationen von üblicherweise $2 \cdot 10^{-4}$ T bis $4 \cdot 10^{-4}$ T über Strahlverlust oder inakzeptable Änderungen der Strahleigenschaften entscheiden, ist die durch Remanenz hervorgerufene Mehrdeutigkeit zwischen Magnetstrom und erzeugtem Magnetfeld in der Regel nicht tolerabel.

Auch bei Teilchenbeschleunigern wurde daher bereits eine Konditionierung der Beschleunigermagnete vorgeschlagen. Ein übliches Konditionierungsverfahren besteht in der Anwendung eines sogenannten "Kamins" (auch als "chimney° bezeichnet). Hier werden die Beschleunigermagnete üblicherweise am Ende eines Teilchenstrahlextraktionszyklus in die Sättigung und anschließend auf einen Nullstrom gefahren. Dadurch werden kontrollierte Ausgangsbedingungen für den nächsten Beschleunigungszyklus geschaffen. Der Nachteil ist offensichtlich: Für das Hochfahren der Magnete wird Energie ohne eigentlichen Nutzen verbraucht. Auch ist für das Hochfahren der Magnete in den Sättigungsbereich (und das anschließende Herunterfahren) Zeit erforderlich. Die erforderliche Zeitspanne wird umso größer, je niedriger die Teilchenenergie beim vorherigen Beschleunigungszyklus ist.

Unter bestimmten Umständen ist es auch möglich, eine Konditionierung durch sogenannte "Trainingszyklen" herbeizuführen. Dazu wird eine definierte Magnetisierungsvorgeschichte der Magnete zur Verfügung gestellt, wobei nicht notwendigerweise ein Hochfahren der Magnete bis in den Sättigungsbereich hinein erfolgen muss. Ein derartiger Ansatz kann geeignet sein, wenn die Teilchenenergie des Teilchenstrahls nur selten geändert werden muss. Wenn jedoch die geforderten Magnetfeldsequenzen von den Konditionierungszyklen abweichen, ist die Verwendung von Trainingszyklen de facto nicht möglich. Da in der Praxis bis zu fünf (zum Teil auch noch mehr) Trainingszyklen erforderlich sind, ginge dann zu viel Energie und Strahlzeit verloren. Gerade bei medizinischen Anwendungen muss die Teilchenenergie jedoch besonders häufig geändert werden. Dies gilt insbesondere bei Rasterscanverfahren zur Therapie von Tumoren.

Eine weitere Quelle von Mehrdeutigkeiten in der Beziehung zwischen Magnetstrom und Magnetfeld sind dynamische Effekte in den Magneten, wenn diese zeitlich schnell veränderliche Magnetfelder erzeugen. Die Ursache sind dabei in erster Linie Wirbelströme. Gegen dynamische Abweichungen ist Konditionieren unwirksam. Da die dynamischen Abweichungen jedoch mit der Zeit abklingen, können die Fehler durch Abwarten verringert werden. Bei einem typischen Synchrotron-Magneten mit einem Joch aus miteinander verklebten Eisenblechen, der in einer Sekunde von Null auf sein Maximalfeld (üblicherweise im Bereich von 1,5 T bis 2 T) verfahren wird, liegt die anfängliche Ablage vom statischen Endfeld bei bis zu $\pm 3 \cdot 10^{-3}$ T. Die anfängliche dynamische Ablage liegt also im Bereich der Remanenzfehler oder ist größer als diese. Die Abkling-Zeitkonstante liegt bei geklebten Blechen üblicherweise im Bereich von 0,3 Sekunden. Bei Magneten aus massivem Eisen kann die Abkling-Zeitkonstante aber auch im Bereich von vielen Sekunden liegen.

Somit fehlt es nach wie vor an einer befriedigenden Möglichkeit für das Ansteuern von Magneten bei Geräten, die hohe und zeitlich veränderliche Magnetfelder benutzen, wie dies beispielsweise bei therapeutischen Synchrotrons oder NMRs der Fall ist.

Somit liegt der Erfindung die Aufgabe zu Grunde, verbesserte Magnetfelderzeugungsvorrichtungen, sowie Anlagen, die zumindest eine derartige Magnetfelderzeugungsvorrichtung aufweisen, vorzuschlagen. Weiterhin liegt der Erfindung die Aufgabe zu Grunde, ein verbessertes Verfahren zu Ansteuerung derartiger Magnetfelderzeugungsvorrichtungen bzw. von Anlagen, die zumindest eine derartige Magnetfelderzeugungsvorrichtung aufweisen, vorzuschlagen.

Es wird vorgeschlagen eine Magnetfelderzeugungsvorrichtung, die wenigstens einen Magnetgap aufweist, welcher der Aufnahme von Gegenständen dient, auf die ein Magnetfeld, das von wenigstens einem Magnetfelderzeugungsmittel der Magnetfelderzeugungsvorrichtung erzeugt wird, einwirkt, mit wenigstens einem Magnetfeldmessmittel zu versehen, welches in einem außerhalb des Magnetgaps befindlichen Messmittelaufnahmebereich angeordnet ist. Dadurch wird es möglich, eine genaue Kenntnis vom aktuellen Zustand des Magnetfeldes zu erlangen. Eine vorherige Kennlinienmessung im Labor kann gegebenenfalls entfallen, oder aber zumindest vereinfacht werden. Besonders vorteilhaft ist, dass durch die direkte Messung des Magnetfelds die Remanenz, und damit der Einfluss der vorherigen Beschleunigungszyklen, auf besonders einfache Weise erfasst, und somit berücksichtigt werden kann. Insbesondere kann gegebenenfalls auf die Verwendung eines "Kamins" oder auf Trainingszyklen verzichtet werden, oder zumindest können diese deutlich eingeschränkt werden. Es ist sogar möglich, Magnetfeldschwankungen, die durch dynamische Effekte verursacht werden, mit Hilfe des Magnetfeldmessmittels zu erfassen und zu berücksichtigen. Der jeweils aktuell gemessene Wert der Magnetfeldstärke kann beispielsweise dazu verwendet werden, dass der Strom, der zur Anregung des Magnetfelds verwendet wird, entsprechend dem aktuellen Messwert des Magnetfeldmessmittels nachgesteuert wird. Dies kann von einer geeigneten Steuervorrichtung durchgeführt werden. Mit dem Magnetfeldmessmittel kann man das Magnetfeld messen, während die Magnetfelderzeugungsvorrichtung aktiv betrieben wird. Das Magnetfeldmessmittel sollte dabei die Stärke des Magnetfelds vorzugsweise mit einer Genauigkeit von $10^{-4}$, besser $10^{-5}$, bezogen auf den Designwert der Magnetfelderzeugungsvorrichtung messen. Das Ausgangssignal des Magnetfeldmessmittels kann dazu verwendet werden, die Ansteuerung der Magnetfelderzeugungsvorrichtung durchzuführen oder

zu beeinflussen. Mit dieser Ansteuerung bzw. Beeinflussung der Magnetfelderzeugungsvorrichtung kann eine besonders schnelle, temperaturstabile und zeitstabile Erzeugung von Magnetfeldern - insbesondere auch von starken Magnetfeldern - mit hoher Genauigkeit ermöglicht werden. Im Falle einer elektrisch betriebenen Magnetfelderzeugungsvorrichtung kann beispielsweise der Strom, mit dem die Magnetfelderzeugungsvorrichtung (bzw. das Magnetfelderzeugungsmittel) angesteuert wird, gesteuert bzw. beeinflusst werden. Anstatt einer bislang üblichen Magnetstromsteuerung kann dadurch eine direkte Magnetfeldsteuerung realisiert werden. Wird mit Hilfe der Magnetfelderzeugungsvorrichtung beispielsweise der Teilchenstrahl eines Synchrotrons beeinflusst (abgelenkt und/oder fokussiert), so können besonders lagegenaue und/oder besonders gut fokussierte Teilchenstrahlen reproduzierbar erzeugt werden. Durch die Anordnung des Magnetfeldmessmittels außerhalb des Magnetgaps ist es möglich, die Messung des Feldes durch das Magnetfeldmessmittel im Rahmen eines regulären Betriebs der Magnetfelderzeugungsvorrichtung, bzw. der Anlage, in der die Magnetfelderzeugungsvorrichtung verwendet wird, durchzuführen. Das Signal kann dabei in einer Magneterzeugungsvorrichtung gemessen werden, die in die Anlage - beispielsweise in ein Synchrotron - eingebaut ist. Die Magnetfeldmessmittel ist also nicht - wie im Stand der Technik - bestenfalls in einer von der Strahlführung des Synchrotrons räumlich getrennten Magnetfelderzeugungsvorrichtung (welche üblicherweise in nicht unerheblicher Entfernung vom eigentlichen Synchrotron angeordnet ist) eingebaut. Bei Verwendung einer Magnetfelderzeugungsvorrichtung mit dem vorgeschlagenen Aufbau kann daher auf gesonderte (Labor-) Messungen (sei es in gesonderten Vorrichtungen und/oder zu gesonderten Zeitpunkten) gegebenenfalls verzichtet werden, oder aber diese können zumindest stark eingeschränkt werden. Selbstverständlich können gesondert ermittelte Messwerte nach wie vor herangezogen werden, beispielsweise um eine "Grobsteuerung" durchzuführen. Eine "Feinnachjustierung" des Magnetfelderzeugungsmittels (oder anderer Einrichtungen, wie beispielsweise zusätzlicher Magnetfelderzeugungsmittel) kann mit Hilfe der vom Magnetfeldmessmittel gelieferten Messwerte erfolgen. Bei den Gegenständen kann es sich sowohl um Gegenstände handeln, die über längere Zeitdauern (z.B. Patienten bei einem Kernspintomographen - "NMR") oder sogar dauerhaft (Beamröhre bei einem Synchrotron) im Magnetgap verbleiben. Es kann sich aber auch um Gegenstände handeln, die nur kurzzeitig im Magnetgap vorhanden sind, wie dies z.B. bei den Teilchen eines Teilchenstrahls bei einem Synchrotron (oder bei einem anderen Teilchenbeschleuniger) der Fall ist. Unter einem Magnetgap ist im Rahmen dieser Schrift nicht nur die lichte Weite zwischen den Begrenzungsflächen der Magnetfelderzeugungsvorrichtung (z.B. den Polschuhen eines Magneten), sondern insbesondere auch die sogenannte Apertur zu verstehen. Bei der Apertur handelt es sich insbesondere um den Bereich, der für die Aufnahme des vom Magnetfeld der Magnetfelderzeugungsvorrichtung zu beaufschlagenden Gegenstands vorgesehen ist ("Beaufschlagungskavität"). In diesem Sinne wäre es also denkbar, dass ein Magnetfeldmessmittel bei einer als Elektromagnet ausgebildeten Magnetfelderzeugungsvorrichtung auf der Stirnfläche eines Polschuhs angeordnet wird. Als von dem Vorschlag mit umfasst kann es gegebenenfalls auch angesehen werden, wenn das bzw. wenigstens ein Magnetfeldmessmittel innerhalb der Vakuumkammer des Synchrotrons (und damit innerhalb des Magnetgaps), jedoch außerhalb des Bereichs, in dem sich der Teilchenstrahl im regulären Betrieb der Anlage befinden kann, angeordnet ist. Unter einer Magnetfelderzeugungsvorrichtung kann im Übrigen die Magnetfelderzeugungsvorrichtung mit, aber auch ohne Zusatzgeräte, die zu deren Funktionstüchtigkeit erforderlich sind, verstanden werden. Im Falle einer Magnetfelderzeugungsvorrichtung mit elektrisch betriebenen Spulen kann also der Magnet als solcher verstanden werden, aber auch die Kombination aus Magnet und einer (regelbaren) Stromversorgungseinrichtung.

Weiterhin wird vorgeschlagen, die Magnetfelderzeugungsvorrichtung derart auszubilden, dass die Magnetfelderzeugungsvorrichtung zumindest zeitweise ein sich zeitlich veränderndes Magnetfeld erzeugt. Insbesondere kann es sich hierbei um eine Magnetfelderzeugungsvorrichtung handeln, die zumindest zeitweise gepulst und/oder in einem Rampenbetrieb betrieben wird, vor allen Dingen, wenn dabei ein besonders breiter Dynamikbereich abgedeckt wird. Hier ist beispielsweise an Magnete für den Einsatz in Teilchenbeschleunigern (insbesondere Synchrotrons) oder Kernspintomographen (NMR) zu denken. Diese müssen oftmals Magnetfelder mit ansteigenden und abfallenden Flanken im Wechsel mit konstanten Plateaus bzw. sogenannte "sweeps" erzeugen. Gerade bei derartigen, zeitlich veränderlichen Feldern können die Vorteile, die sich durch eine genauere Kenntnis von der Stärke des Magnetfeldes ergeben können, besonders groß sein. Insbesondere ist es auch möglich, dynamische Effekte, die bei derartigen, zeitlich variierenden Magnetfeldern auftreten können, berücksichtigen zu können. Bei den Magneten für Teilchenbeschleuniger ist speziell an Strahlführungsmagnete (Dipole), als auch an Strahlfokussierungsmagnete (Quadrupole) zu denken.

Möglich ist es, dass die Magnetfelderzeugungsvorrichtung derart ausgebildet ist, dass die Magnetfelderzeugungsvorrichtung zumindest eine Dipolmagnetfelderzeugungseinrichtung und/oder zumindest eine Quadrupolmagnetfelderzeugungseinrichtung aufweist. Dabei kann es sich insbesondere um elektrisch betriebene Dipolmagnete und/oder elektrisch betriebene Quadrupolmagnete handeln, die bevorzugt als Magnete mit normalleitenden Stromspulen ausgebildet sind. Insbesondere können die Magnete bzw. Magnetfelderzeugungsvorrichtungen zumindest ein Joch bzw. zumindest einen Magnetkern aus einem weichmagnetischen Material aufweisen. Derartige Magnetfelderzeugungseinrichtungen sind insbesondere bei Kernspintomographen (NMRs) und Teilchenbeschleunigern, wie insbesondere Synchrotrons erforderlich. Bei einem Synchrotron dienen beispielsweise Dipolmagnetfelderzeugungseinrichtungen der Strahlablenkung, während Quadrupolmagnetfelderzeugungseinrichtungen der Strahlfokussierung dienen. Da manche Magnetfelderzeugungsvorrichtungen bei einem Synchrotron sehr häufig (z.B. mehrere hunderttausend Mal) vom gleichen Teilchenstrahlpaket durchlaufen werden, ist es sehr vorteilhaft, wenn die entsprechenden Magnetfelderzeugungsvorrichtungen sehr genaue Magnetfelder erzeugen. Denn bereits relativ kleine Fehler können sich über mehrere Durchläufe hinweg aufsummieren und zu einem Verlust des Teilchenstrahls bzw. zu einer derartigen Verschlechterung des Teilchenstrahls führen, sodass dieser letztendlich nicht mehr verwendbar ist. Denkbar sind aber auch davon abweichende Magnetfelderzeugungsvorrichtungen, wie insbesondere Magnetfelderzeugungsvorrichtungen mit 2·n Polen, wobei insbesondere n=3, 4, 5 oder 6 gelten kann.

Ein möglicher Aufbau für eine Magnetfelderzeugungsvorrichtung ergibt sich, wenn zumindest ein Magnetfeldmessmittel als Induktionsmessmittel, insbesondere als Leiterschleife ("pick-up coil") und/oder als Spule ausgebildet ist. Derartige Magnetfeldmessmittel benötigen üblicherweise nur einen sehr kleinen Bauraum. Darüber hinaus können derartige Magnetfeldmessmittel üblicherweise auch besonders einfach und flexibel an einen verwinkelten Bauraum angepasst werden. Weiterhin sind derartige Magnetfeldmessmittel in der Regel auch kostengünstig, weisen üblicherweise keine bzw. keine bedeutende Temperaturdrift auf, und sind meist auch besonders lange verwendbar, ohne dass es zu nennenswerten Alterungserscheinungen kommt. Ein weiterer Vorteil derartiger Magnetfeldmessmittel ist es, dass diese in der Regel sehr schnell messen können. Gerade dann, wenn das vom Magnetfeldmessmittel erzeugte Messsignal (auch) für eine Rückkopplung zur Nachsteuerung der Ansteuerung des Magnetfelderzeugungsmittels verwendet werden soll, ist eine schnelle Messung üblicherweise von großer Wichtigkeit. Schließlich sind die vorgeschlagenen Magnetfeldmessmittel auch für Umgebungen verwendbar, bei denen ein stärkerer elektromagnetischer Untergrund-Rauschpegel (electromagnetic background noise) vorherrscht. Bei einem derartigen elektromagnetischen Untergrund-Rauschpegel können beispielsweise Hallsonden Probleme aufweisen, da sie funktionsbedingt mit einem möglichst konstanten Gleichstrom versorgt werden müssen. Dieser Gleichstrom muss jedoch in der Regel über relativ lange Stromzuführungsleitungen dem Messort zugeführt werden. Dabei kann der elektromagnetische Untergrund-Rauschpegel in das Stromkabel eingekoppelt werden, wodurch sich die Genauigkeit der Messwerte verschlechtern kann. Ein weiter Vorteil einer Leiterschleife bzw. einer Spule kann darin bestehen, dass diese das Feldintegral über die Leiterschleifenfläche bzw. Spulenfläche misst. Ein derartiger "flächiger" Messwert kann punktuellen Messwerten, wie sie beispielsweise von einer Hallsonde oder einer magnetoresistiven Sonde geliefert werden, überlegen sein. Insbesondere ist es möglich, dass man bei der Anordnung des Magnetfeldmessmittels weniger genau auf die Positionierung des Magnetfeldmessmittels achten muss. Dies kann speziell bei inhomogenen Feldern zu einer unter Umständen deutlichen Verbesserung der Messgenauigkeit führen. Im Falle von Synchrotrons kann dies zu einer deutlichen Verbesserung der Lage und/oder der Fokussierung des Teilchenstrahls führen. Das Induktionsmessmittel kann bevorzugt als starr angeordnete Spule bzw. Leiterschleife ausgebildet sein. Es ist jedoch auch denkbar, dass das Induktionsmessmittel auch eine drehbare Spule bzw. Leiterschleife aufweist, die insbesondere mit Hilfe einer Antriebseinrichtung, wie einem Elektromotor, in eine Drehbewegung versetzt werden kann.

Es kann sich als vorteilhaft verweisen, wenn eine Magnelfelderzeugungsvorrichtung, insbesondere eine Magnetfelderzeugungsvorrichtung, welche ein Induktionsmessmittel aufweist, wenigstens eine Integratoreinrichtung aufweist. Übliche Induktionsmessmittel können nur die zeitliche Veränderung des Magnetfeldes messen, also $A \propto dB/dt$, wobei $A$ der Messwert des Magnetfeldmessmittels ist. Mit anderen Worten kann das vorhandene Magnetfeld nur über eine zeitliche Integration $B = \int A\, dt$ (ausgehend von einem Startwert) ermittelt werden. Dies kann mit Hilfe der Integratoreinrichtung erfolgen, wobei diese als analoge Integratoreinrichtung, als digitale Integratoreinrichtung oder als Kombination aus analoger und digitaler Integratoreinrichtung ausgebildet sein kann.

Weiterhin ist es möglich, eine Magnetfelderzeugungsvorrichtung derart auszubilden, dass zumindest ein Magnetfeldmessmittel im Bereich zumindest eines Polschuhs und/oder zumindest eines Jochs angeordnet ist. Versuche haben ergeben, dass die dort gemessenen Werte in der Regel am besten mit dem Magnetfeld korrelieren, das von den in dem Magnetgap vorhandenen Gegenständen, bzw. von den durch den Magnetgap hindurchgeführten Gegenständen "gespürt" wird. Die Kenntnis über das "effektiv" anliegende Magnetfeld kann somit nochmals genauer werden. Möglich ist es insbesondere, dass wenigstens ein Magnetfeldmessmittel als Leiterschleife ausgebildet ist, die den jeweiligen Polschuh zumindest im Wesentlichen vollständig umgreift. Im Zusammenhang mit einem Synchrotron (bzw. ganz allgemein im Zusammenhang mit einem Teilchenstrahl) ist es möglich, die Messung quasi auf den virtuellen Pfad des Teilchenstrahls zu "fokussieren". Dies gilt insbesondere, da dieser üblicherweise symmetrisch im Magnetgap, zu den Polschuhen und/oder dem Joch angeordnet ist. Dadurch kann der Teilchenstrahl besonders ortsgenau geführt werden und/oder besonders fein fokussiert werden.

Eine weitere vorteilhafte Weiterbildung der Magnetfelderzeugungsvorrichtung kann sich ergeben, wenn diese zumindest ein direktes Magnetfeldmessmittel aufweist, welches insbesondere als NMR-Sonde, als magnetoresistives Messelement und/oder als Hallsonde ausgebildet ist. Unter einem "direkten" Magnetfeldmessmittel ist insbesondere ein Messmittel zu verstehen, welches die Größe des Magnetfeldes "direkt" und unmittelbar (vorzugsweise auch absolut) messen kann, und nicht nur mittelbar basierend auf der zeitlichen Veränderung des Magnetfeldes. Dadurch können insbesondere die Offset-Probleme, die sich bei einer Integration eines Messsignals ergeben können, vermieden werden. Besonders vorteilhaft kann es sein, wenn derartige direkte Magnetfeldmessmittel zusätzlich zu Induktionsmessmitteln vorgesehen werden. Insbesondere Hallsonden sollten in der Regel nur als zusätzliche Magnetfeldmessmittel (z.B. zusätzlich zu einer Leiterschleife) eingesetzt werden. Die Magnetfeldmessmittel können dann beispielsweise dazu verwendet werden, um die sich mit der Zeit ergebenden Offset-Driften der Induktionsmessmittel (bzw. der dazugehörigen Integratoreinrichtungen) zu kompensieren. Unter einem magnetoresistiven Messelement sind insbesondere solche Messelemente zu verstehen, welche einen magnetoresistiven Werkstoff (giant magneto-resistivity) aufweisen. Gegebenenfalls kann auch eine drehbar gelagerte und angetriebene Spule bzw. Leiterschleife als direktes Magnetfeldmessmittel genutzt werden. Dies gilt insbesondere dann, wenn die Richtung des Magnetfelds konstant ist, bzw. (ggf. durch weitere Messmethoden) bekannt ist. Kennt man zusätzlich noch die jeweilige Winkelstellung der rotierenden Spule bzw. Leiterschleife kann die Messung der Stärke des Magnetfelds beispielsweise ausgehend von einer Parallelstellung von Spulennormaler und Magnetfeldlinien während der darauffolgenden 90°-Drehung der Spule erfolgen. Die Rotationsfrequenz der Spule sollte dabei vorzugsweise schnell im Verhältnis zur Geschwindigkeit der Magnelfeldänderung sein. Eine NMR-Sonde ist in der Regel nur für Dipolmagnetfelderzeugungsvorrichtungen verwendbar, da NRM-Sonden üblicherweise Probleme mit inhomogenen Feldern haben, wie diese beispielsweise bei Quadrupolmagnetfelderzeugungsvorrichtungen auftreten.

Insbesondere ist es möglich, die Magnetfelderzeugungsvorrichtung derart auszubilden, dass zumindest ein Magnetfeldmessmittel als Triggereinrichtung verwendet wird. Insbesondere kann der Messwert eines direkten Messmittels als "Nullstellungstrigger" für ein Induktionsmessmittel (bzw. für die in Zusammenhang mit dem Induktionsmessmittel verwendete Integratorvorrichtung) verwendet werden. Dieser Ausbildung liegt die Erkenntnis zu Grunde, dass direkte Magnetfeldmessmittel, die auch starke Magnetfelder quantitativ messen können, oftmals schnell altern bzw. starken Temperaturschwankungen unterliegen, teuer sind, oder aber bei Bereitstellung eines hinreichend großen und genauen Messbereichs relativ groß sind. Werden dagegen direkte Magnetfeldmessmittel nur dazu verwendet, den Nulldurchgang des Magnetfeldes zu ermitteln, so können diese meist deutlich einfacher aufgebaut werden und weisen üblicherweise deutlich weniger der sonstigen genannten Nachteile auf. Um eine Nulliniendrift des Induktionsmessmittels (bzw. der Integratoreinrichtung) zu verhindern, reichen derartige einfache direkte Magnetfeldmessmittel jedoch üblicherweise vollauf aus. Das Gesagte gilt dabei nicht nur für einen Nulldurchgang des Magnetfeldes, sondern in analoger Weise auch für einen Durchgang bei einem (oder bei mehreren) definierten Werte der Magnetfeldstärke und/oder bei einem Durchgang der Magnetstromstärke durch einen bestimmten Wert (wie beispielsweise ein Magnetstromnulldurchgang). Beispielsweise könnte ein direktes Magnetfeldmessmittel, wie beispielsweise eine Hallsonde, auf eine Magnetfeldstärke optimiert werden, bei der die Injektion des Teilchenstrahls in ein Synchrotron hinein erfolgt. Da die Injektion des Teilchenstrahls (oder auch andere Plateauphasen bei einem Beschleunigungszyklus) eine gewisse Zeit dauert, ist es gegebenenfalls möglich, auch direkte Magnetfeldmessmittel zu verwenden, die eine gewisse Zeitdauer für die Messung benötigen, wie beispielsweise NMR-Sonden.

Weiterhin kann es sich als vorteilhaft erweisen, wenn bei einer Magnetfelderzeugungsvorrichtung zumindest ein Messsignal zumindest eines Magnetfeldmessmittels als Eingangssignal zur Ansteuerung wenigstens eines Magnetfelderzeugungsmittels der Magnetfelderzeugungsvorrichtung verwendet wird. Mit einer derartigen Ausbildung der Magnetfelderzeugungsvorrichtung ist es nicht nur möglich, das tatsächlich vorhandene Magnetfeld zu kennen, sondern die somit gewonnene Erkenntnis zur Stabilisierung des Magnetfeldes zu verwenden. So ist es beispielsweise möglich, den Strom, mit dem zumindest eines der Magnetfelderzeugungsmittel der Magnetfelderzeugungsvorrichtung beaufschlagt wird, so nachzusteuern, dass sich das gewünschte Magnetfeld ergibt. Somit ist es möglich, der Magnetfelderzeugungsvorrichtung als Sollwert eine bestimmtes Magnetfeld vorzugeben, und nicht mehr - wie bislang üblich - einen gewissen Stromwert. Zur Ansteuerung wenigstens eines Magnetfelderzeugungsmittels der Magnetfelderzeugungsvorrichtung kann insbesondere eine entsprechend ausgebildete Steuerungseinrichtung verwendet werden. Dabei kann eine gesonderte Steuerungseinrichtung verwendet werden (beispielsweise ein Einplatinencomputer), oder eine bereits vorgesehene Steuerungseinrichtung kann diese Aufgabe zusätzlich übernehmen. Denkbar wäre es beispielsweise eine DCCT-Steuereinrichtung geeignet zu modifizieren.

Weiterhin wird eine Anlage mit wenigstens einer Magnetfelderzeugungsvorrichtung vorgeschlagen, welche wenigstens eine Magnetfelderzeugungsvorrichtung mit dem oben vorgeschlagenen Aufbau aufweist. Das entsprechende Gerät weist dann die bereits beschriebenen Eigenschaften und Vorteile in analoger Weise auf.

Insbesondere ist es möglich, die Anlage derart auszubilden, dass sie ein Synchrotron umfasst, wobei das Synchrotron zumindest eine Magnetfelderzeugungsvorrichtung aufweist, welche als Dipolmagnetfelderzeugungsvorrichtung und/oder als Quadrupolmagnetfelderzeugungsvorrichtung ausgebildet ist, wobei die zumindest eine Magnetfelderzeugungsvorrichtung zumindest ein Magnetfeldmessmittel umfasst, das in einem Magnetgap der zumindest einen Magnetfelderzeugungsvorrichtung angeordnet ist, wobei der Messwert des zumindest einen Magnetfeldmessmittels zur Ansteuerung und/oder Nachregelung zumindest einer Magnetfelderzeugungsvorrichtung, insbesondere zur Ansteuerung der Magnetfelderzeugungsvorrichtung, in dem das zumindest eine Magnetfeldmessmittel angeordnet ist, verwendet wird. Die Dipolmagnetfelderzeugungsvorrichtungen dienen dabei in erster Linie der Strahlablenkung, während die Quadrupolmagnetfelderzeugungsvorrichtungen in erstere Linie der Strahlfokussierung des Teilchenstrahls des Synchrotrons dienen. Mit den Quadrupolmagnetfelderzeugungsvorrichtungen kann also die Form des Ionenstrahls (rund, oval, punktförmig, flächenförmig usw.) eingestellt werden. Sowohl die Ablenkung des Teilchenstrahls, als auch dessen Fokussierung müssen gerade für medizinische Anwendungen - wie beispielsweise bei der Tumortherapie - genau reproduzierbar sein. Größere Ablenkungen können nicht nur die Schädigung von nicht zu bestrahlendem Gewebe verursachen, sondern können auch zum Strahlverlust führen. Der mit Hilfe des Synchrotrons erzeugte Ionenstrahl wird in der Regel durch Ablenkmagnete in zwei zueinander senkrechten Richtungen abgelenkt (x-Richtung, $\gamma$-Richtung) und dem zu bestrahlenden Gebiet (Tumor) zugeführt. Die Tiefenvariation (z-Richtung) kann durch passende Einstellung der Teilchenenergie erfolgen. Die Energie wird dabei üblicherweise so gewählt, dass der Teilchenstrahl seine Hauptenergie (aufgrund des sogenannten Bragg-Peaks) im Bereich des Zielgebiets (Tumor) verliert. Insbesondere beim sogenannten Rasterscanning-Verfahren, bei dem der Ionenstrahl das Zielgebiet punkt- bzw. zeilenartig (gegebenenfalls auch mehrfach) abtastet, muss die Energie des Ionenstrahls (aufgrund der variierenden Behandlungstiefe) in der Regel besonders häufig variiert werden. Dementsprechend muss auch die Ansteuerung der Magnetfelderzeugungsvorrichtungen des Synchrotrons variiert werden. Trotz der sich dabei ändernden Teilchenenergien sollte die Ortlage und Fokussierung des Ionenstrahls reproduzierbar einstellbar sein. Dies kann auf vorteilhafte Weise durch die genaue (z.B. besser als $10^{-4}$, bevorzugt besser als $10^{-5}$ vom Designwert) Einstellbarkeit der Magnetfelderzeugungsvorrichtungen des Synchrotrons erfolgen. Dies kann dadurch ermöglicht werden, dass die Magnetfelderzeugungsvorrichtungen unter Verwendung eines Messsignals, dass wenigstens eine Magnetfeldmesseinrichtung erzeugt, geregelt werden. Dieses Messsignal kann ein Ausgangssignal zur Ansteuerung einer Stromquelle der Magnetfelderzeugungsvorrichtung (bzw. des Magnetfelderzeugungsmittels, wie beispielsweise einer normalleitenden Spule der Magnetfelderzeugungsvorrichtung) beeinflussen.

Als besonders vorteilhaft kann es sich erweisen, wenn die Anlage zumindest teilweise als medizinische Therapieeinrichtung, als medizinische Diagnoseeinrichtung, als Computertomograph, als Kernspintomograph (NMR) und/oder als Teilchenbeschleuniger, insbesondere als Linearbeschleuniger und/oder als Synchroton ausgebildet ist. Gerade bei derartigen Geräten machen sich die Vorteile, die durch den oben vorgeschlagenen Aufbau entstehen können, in der Regel besonders stark bemerkbar. Selbstverständlich kann die Anlage auch (primär) nicht-medizinischen Zwecken dienen. Beispielsweise ist auch eine zumindest teilweise Ausbildung der Anlage als Experimentierspeicherring, als Experimentierlinearbeschleuniger und/oder als Spektrometer denkbar.

Weiterhin wird ein Verfahren zum Betrieb einer Magnetfelderzeugungsvorrichtung und/oder einer Anlage mit wenigstens einer Magnetfelderzeugungsvorrichtung, insbesondere ein Verfahren zum Betrieb einer Magnetfelderzeugungsvorrichtung mit dem oben vorgeschlagenen Aufbau und/oder einer Anlage mit dem oben vorgeschlagenen Aufbau, vorgeschlagen, bei dem während des Betriebs der Magnetfelderzeugungsvorrichtung das von dem Magnetfeldmessmittel gemessene Magnetfeld zur Ansteuerung zumindest eines Magnetfelderzeugungsmittels der Magnetfelderzeugungsvorrichtung, insbesondere zur Ansteuerung einer Stromversorgungsvorrichtung des Magnetfelderzeugungsmittels verwendet wird. Mit einem derartigen Verfahren ist es insbesondere möglich, die Messwerte bezüglich der Größe des tatsächlich vorherrschenden Magnetfelds zur Stabilisierung des Magnetfeldes zu verwenden. So ist es beispielsweise möglich, den Strom, mit dem zumindest eines der Magnetfelderzeugungsmittel der Magnetfelderzeugungsvorrichtung beaufschlagt wird, so nachzusteuern, dass sich das gewünschte Magnetfeld ergibt. Somit ist es möglich, der Magnetfelderzeugungsvorrichtung eine bestimmte Magnetfeldstärke als Sollwert vorzugeben, und nicht mehr - wie bislang üblich - einen gewissen Stromwert

Insbesondere kann das Verfahren derart durchgeführt werden, dass die Ansteuerung zumindest eines Magnetfelderzeugungsmittels zumindest zeitweise und/oder zumindest bereichsweise und/oder zumindest teilweise in Abhängigkeit von dem von dem Magnetfeldmessmittel gemessenen Magnetfeld erfolgt. Dies entspricht in aller Regel einer direkten Vorgabe des Magnetfelds, das anschließend in dieser Stärke erzeugt wird. Diese Vorgabe kann auch nur für bestimmte Bereiche, beispielsweise für das Magnetgap, erfolgen. Auch ist es denkbar, dass die Vorgabe eines bestimmten Magnetfeldes nur zu bestimmten Zeitpunkten, beispielsweise bei den ansteigenden und/oder abfallenden Rampen bzw. beim Erreichen des Extraktionsplateaus erfolgt. Auch kann eine gemischte Regelung aus Stromstärke und Magnetfeld erfolgen.

Insbesondere ist es möglich, das Verfahren derart durchzuführen, dass die Ansteuerung zumindest zeitweise und/oder zumindest bereichsweise und/oder zumindest teilweise gemäß einem vorab ermittelten Ansteuerungsmodell erfolgt, welches zumindest zeitweise und/oder zumindest bereichsweise und/oder zumindest teilweise in Abhängigkeit von dem von dem Magnetfeldmessmittel gemessenen Magnetfeld nachgeführt wird. Dadurch ist es beispielsweise möglich, eine maximale Stromänderung pro Zeiteinheit vorzugeben, um so gewisse Komponenten der Stromverstärkerschaltungen nicht zu überlasten. Auch ist es möglich, die Schaltung so auszuführen, dass bei einem Ausfall der Magnetfeldmessmittel nach wie vor ein "klassischer" Betrieb der Anlage möglich ist. Die Anlage kann dann bis zur Reparatur weiter betrieben werden, wenn auch mit verschlechterter Effizienz. Eine derartige erhöhte Betriebssicherheit ist insbesondere bei medizinischen Anwendungen von großem Wert.

Im Folgenden wird die Erfindung anhand vorteilhafter Ausführungsbeispiele und unter Bezugnahme auf die beigefügte Zeichnung näher erläutert. Es zeigt:

Fig. 1:      einen Dipolmagneten für ein Synchrotron in schematischer Draufsicht von vorne;

Fig. 2:      einen schematischen Querschnitt durch den in Fig. 1 dargestellten Dipolmagneten;

Fig. 3:      einen Quadrupolmagneten für ein Synchrotron in schematischer Draufsicht von vorne;

Fig. 4:      einen schematischen Querschnitt durch den in Fig. 3 dargestellten Quadrupolmagneten;

Fig. 5:      einen Schaltplan für eine Steuerschaltung für einen Magneten;

Fig. 6:      ein Beispiel für das Verhalten eines Magneten mit modellbasierter Ansteuerung;

Fig. 7:      ein Beispiel für das Verhalten eines Magneten mit Magnetfeldansteuerung;

Fig. 8:      ein Ausführungsbeispiel für ein Magnetfeldregelungsverfahren;

Fig. 9:      ein Ausführungsbeispiel für einen Aufbau zur Kalibrierung einer Messschleife eines Magneten;

Fig. 10:      ein Ausführungsbeispiel für eine Therapieanlage.

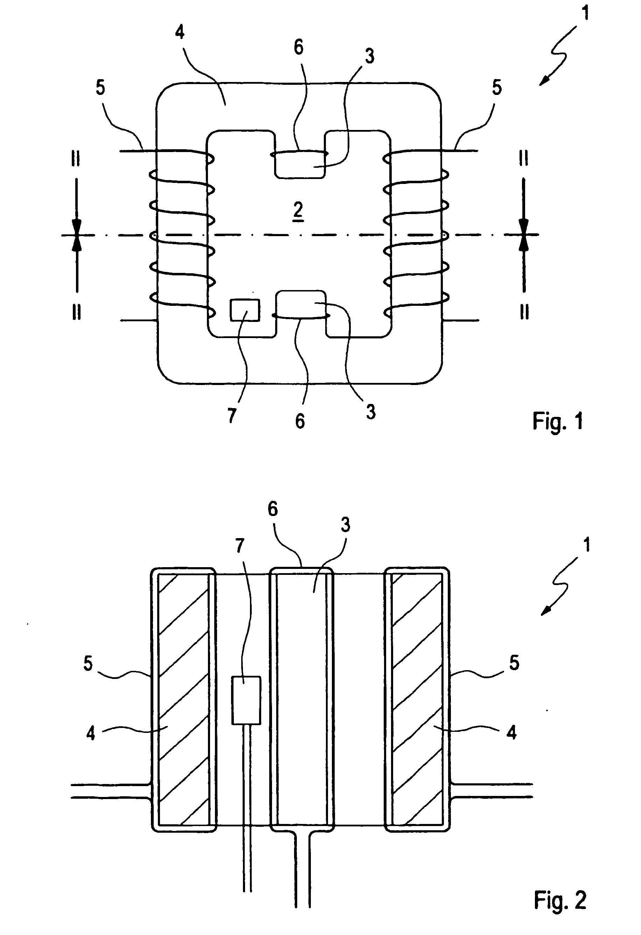

Fig. 1 zeigt in einer schematischen Draufsicht ein Ausführungsbeispiel für einen Dipolmagneten 1 für ein Synchrotron. Derartige Dipolmagneten 1 werden in Synchrotrons dazu verwendet, um den Teilchenstrahl abzulenken, und dadurch auf eine Umlaufbahn zu bringen. In der Mittelausnehmung 2 des Dipolmagneten 1 ist normalerweise das Strahlführungsrohr angeordnet, in dem ein Vakuum herrscht und in dem der Teilchenstrahl verläuft.

Unmittelbar benachbart zur Mittelausnehmung 2 sind die beiden Polschuhe 3 des Dipolmagneten 1 zu erkennen. Die Polschuhe 3 sind über ihre der Mittelausnehmung 2 abgewandte Rückseite über ein Joch 4 aus einem weichmagnetischen Material miteinander verbunden. Um das Joch 4 ist im in Fig. 1 dargestellten Ausführungsbeispiel auf beiden Seiten eine Spule 5 herumgewickelt. Bei einer Beaufschlagung der Spulen 5 mit elektrischem Strom wird somit in der Mittelausnehmung 2 ein Magnetfeld erzeugt.

Weiterhin ist in Fig. 1 im Bereich der Polschuhe 3 jeweils eine Messschleife 6 vorgesehen. Unter Verwendung dieser Messschleifen 6 kann das Magnetfeld, das in der Mittelausnehmung 2 herrscht, sehr genau ermittelt werden. Dabei haben Versuche ergeben, dass es in der Regel ausreichend ist, wenn die Messschleife 6 nur an einem Polschuh 3 vorgesehen wird. Grundsätzlich ist es natürlich auch möglich, zusätzlich oder alternativ eine oder mehrere Messschleifen 6 im Bereich des Jochs 4 des Dipolmagneten 1 vorzusehen.

Da die Messschleifen 6 aufgrund ihres Messprinzips nur zeitliche Änderungen von Magnetfeldern erfassen können, kann die Stärke des Magnetfeldes von den Messschleifen 6 nicht direkt erfasst werden. Zur Bestimmung der Stärke des Magnetfelds wird daher ein zusätzlicher Integrator vorgesehen, der die von der oder von den Messschleifen 6 stammenden Signale über die Zeit integriert, und somit einen Rückschluss auf die Stärke des Magnetfelds erlaubt. Dabei ist es möglich, sowohl analoge, als auch numerische Integratoren einzusetzen.

Beispielsweise kann ein gewöhnlicher ADC (analog to digital converter) zur Integration verwendet werden. Der Ausgabewert $B_i$ des ADC wird beispielsweise in festen Zeitintervallen $\Delta t$ ausgewertet. Die Feldstärke lässt sich dann aus dem Startwert $c$ der Integration plus der Summe aller ADC-Messwerte multipliziert mit $\Delta t$ ermitteln. Es gilt $B(t) = c + \sum_i B_i \Delta t$ .

In ersten Versuchen hat sich jedoch ergeben, dass die Verwendung eines U/f-Wandlers (Spannungs-Frequenz-Wandlers) vermutlich bessere Ergebnisse liefert. Das Spulensignal wird dabei dem U/f-Wandler zugeführt. Jeder vom U/f-Wandler abgegebene Impuls entspricht dann einem definierten Feldstärkeinkrement $\Delta B$. Die Pulse können von einem Zähler aufaddiert werden. Die Gesamtzahl $n_i$ der Pulse entspricht dann der aktuellen Gesamt-Feldstärkeänderung $dB_i$ (wobei noch eine additive Konstante $c$ zu berücksichtigen ist). Es gilt

$$B(t) = c + \sum_i n_i dB_i .$$

Unabhängig von der Art des verwendeten Integrators kann jedoch bei der (ausschließlichen) Verwendung von Messschleifen 6 eine Drift der Nulllinie bei der Bestimmung des Magnetfelds (additive Konstante) grundsätzlich nicht ausgeschlossen werden.

Um auch die Nulliniendrift in den Griff zu bekommen, können zusätzliche Maßnahmen ergriffen werden. So ist es beispielsweise möglich, eine Nachjustage der ausgegebenen Magnetfeldstärke vorzusehen. Um Streuschwankungen zu vermeiden, können die zur Nachjustage verwendeten Signale über mehrere Zyklen gemittelt werden und/oder mehrere Signale zur Nachjustage verwendet werden. Als Eingangssignal für die Nachjustage kann beispielsweise der Nulldurchgang der angelegten Spannung verwendet werden. Eine andere Möglichkeit besteht darin, zusätzliche Messsensoren 7 vorzusehen, welche beispielsweise dazu verwendet werden, das Vorliegen eines Nulldurchgangs des Magnetfelds (oder eines sehr kleinen Magnetfeldes) zu detektieren. Auch ist es möglich, den Teilchenstrahl selbst als Justagesignal zu verwenden. Weist der (extrahierte) Strahl bestimmte Eigenschaften auf, so erfolgt eine Nachjustage.

Ganz allgemein ist darauf hinzuweisen, dass der Ausgabewert nicht unbedingt den Rückschluss auf einen Absolutwert des Magnetfeldes ermöglichen muss. Vielmehr reicht eine Kalibrierung, bei der der Teilchenstrahl als Eichnormal verwendet wird. Hat der Strahl die gewünschten Eigenschaften, kann man die Magnetfeldmessung über den ganzen Zyklus protokollieren. Dies kann dann die Sollwerttabelle darstellen. Weicht in einem späteren Beschleunigungszyklus der Magnetfeldverlauf von der Sollwerttabelle ab, etwa weil der Beschleuniger andere Sequenzen fährt, so ist es die Aufgabe des Feldreglers, den Istwert des Magnetfeldes auf den Sollwert zu bringen und dem Teilchenstrahl reproduzierbar die gewünschten Eigenschaften zu geben. Dazu kann beispielsweise der Strom, der durch die Spulen 5, die das Magnetfeld erzeugen, fließt, entsprechend nachreguliert werden. Mit anderen Worten erfolgt eine Magnetfeldregelung basierend auf einer "in situ"-Kalibrierung.

Bei dem in Fig. 1 dargestellten Ausführungsbeispiel eines Dipolmagneten 1 ist zu Nachjustagezwecken ein zusätzlicher Sensor 7 seitlich benachbart zu einem der Polschuhe 3 des Dipolmagneten 1 angeordnet. Der Sensor 7 liegt somit ebenfalls außerhalb der Mittelausnehmung 2. Dabei ist der zusätzliche Sensor 7 vorliegend als Hallsonde 7 ausgebildet. Da die Hallsonde lediglich zur "Nullkalibrierung" des Magnetfeldmesswertes verwendet wird, ist die Anordnung der Hallsonde 7 in weiten Bereichen variabel. Insbesondere ist es nicht erforderlich, dass die Hallsonde 7 einen genauen Absolutwert (abgesehen vom - im vorliegenden Ausführungsbeispiel - Nulldurchgang) liefert. Die Hallsonde kann also auch in einem Bereich angeordnet werden, der nicht unbedingt repräsentativ für die integrale Feldverteilung sein muss. Es ist vielmehr in der Regel ausreichend, wenn eine "in situ"-Kalibrierung durchgeführt werden kann. Vorzugsweise erfolgt die "Nullkalibrierung" bei einem Zwischenflattop des Magnetfeldes, beispielsweise beim Teilcheninjektionsflattop. Da das Magnetfeld bei einem Zwischenflattop einige Zeit bei einer relativ konstanten Magnetfeldstärke verharrt, kann die Messung ggf. auch etwas langsamer erfolgen. Dadurch können langsamere Messsensoren bzw. Messverfahren genutzt werden. Auch ist es möglich Messsensoren zu verwenden, die ihre Genauigkeit z.B. erst durch numerisches postprocessing erhalten (was üblicherweise eine gewisse Zeitdauer für die dazu erforderlichen Berechnungen erfordert). Dies ist beispielsweise bei kommerziell erhältlichen, hochgenauen Hallsonden bzw. bei kommerziell erhältlichen, hochgenauen magnetoresistiven Sonden der Fall.

In Fig. 2 ist der in Fig. 1 gezeigte Dipolmagnet 1 in einer schematischen Draufsicht von einer Querschnittsfläche II-II aus gesehen zu erkennen. Der Schnitt verläuft dabei längs der in Fig. 1 eingezeichneten Ebene II-II. Gut ist in der Querschnittsansicht zu erkennen, dass die Leiterschleife 6 den gesamten Polschuh 3 des Dipolmagneten 1 umgibt. Die Messschleife 6 registriert somit das gesamte Magnetfeld, das auf die Teilchen des Teilchenstrahls beim Durchlaufen des Dipolmagneten 1 einwirkt.

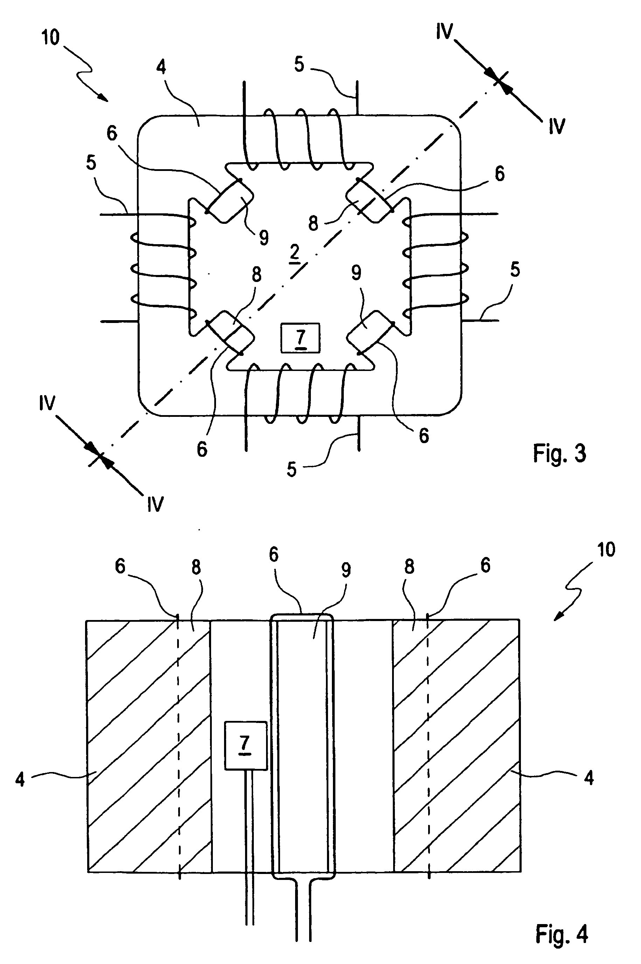

In Fig. 3 sind die Verhältnisse bei einem Quadrupolmagneten 10 dargestellt. Quadrupolmagneten 10 werden in Synchrotrons zur Strahlfokussierung des Teilchenstrahls verwendet. Ein Quadrupolmagnet 10 weist insgesamt vier Polschuhe 8, 9 auf, nämlich jeweils zwei Polschuhe 8, 9 mit gleicher Polarität. Die Polschuhe 8, 9 mit gleicher Polarität sind dabei jeweils einander gegenüberliegend angeordnet. Zur Strahlfokussierung in x- und $\gamma$-Richtung (Ebene senkrecht zur Bewegungsrichtung der Teilchen) müssen dabei mindestens zwei Quadrupolmagneten 10 verwendet werden, welche um 90° zueinander verdreht sind.

Auch im in Fig. 3 dargestellten Ausführungsbeispiel ist um die Polschuhe 8, 9 des Quadrupolmagneten 10 jeweils eine Messschleife 6 gewickelt. Versuche haben ergeben, dass es üblicherweise ausreicht, wenn nur zwei der vier Polschuhe 8, 9 mit einer Messschleife 6 versehen werden. Besonders gute Messergebnisse ergeben sich insbesondere, wenn zwei Polschuhe 8, 9 mit jeweils gleicher Polarität (magnetischer Nordpol bzw. magnetischer Südpol) mit einer Messschleife 6 versehen werden. Es kann sich aber auch als ausreichend erweisen, wenn lediglich ein einziger Polschuh 8, 9 mit einer Messschleife 6 versehen wird. Weiterhin ist - analog zum in Fig. 1 dargestellten Dipolmagneten 1 - auch vorliegend eine Hallsonde 7 vorgesehen.

In Fig. 4 ist eine schematische Draufsicht auf den in Fig. 3 gezeigten Quadrupolmagneten 10 dargestellt, wobei die Draufsicht von der in Fig. 3 eingezeichneten Querschnittsebene IV-IV aus erfolgt.

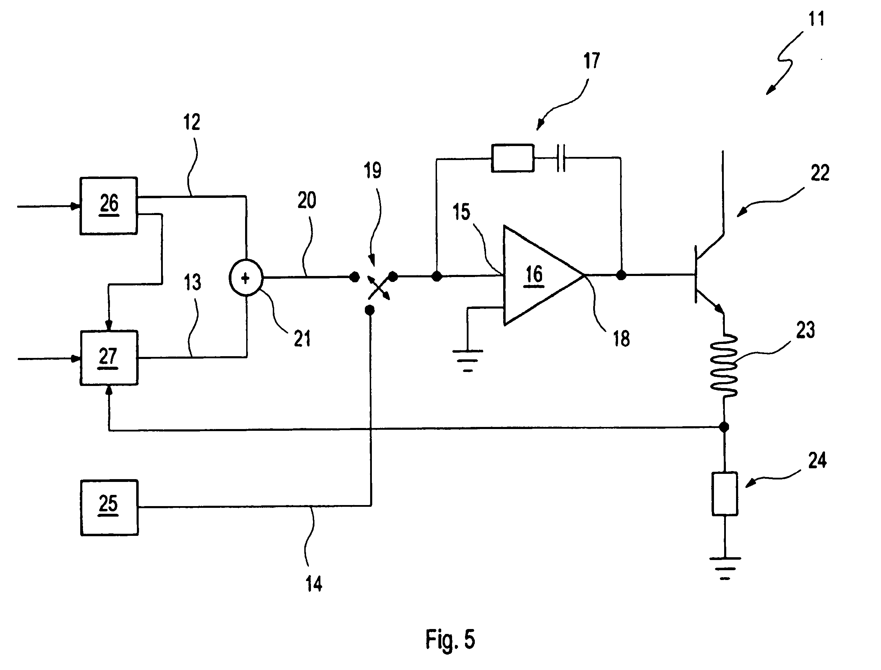

In Fig. 5 ist ein Ausführungsbeispiel für eine elektronische Schaltungsanordnung 11 dargestellt, um die mit Hilfe der Messschleifen 6 und der Hallsonde 7 ermittelte Stärke des Magnetfelds zur Ansteuerung eines Magneten (beispielsweise des Dipolmagneten 1 oder des Quadrupolmagneten 10) zu verwenden.

Der Ausgang 18 des Operationsverstärkers 16 steuert eine Leistungsverstärkungsstufe 22 an, die eine oder mehrere Erregungsspulen 5, 23 eines Dipolmagneten 1 oder eines Quadrupolmagneten 10 mit elektrischem Strom versorgt.

Der nicht-invertierende Eingang 15 des Operationsverstärkers 16 wird mit Hilfe eines Schalters 19 wahlweise mit zwei unterschiedlichen Eingangssignalen 14, 20 verbunden. Weiterhin ist der Ausgang 18 des Operationsverstärkers 16 über einen Hochpass 17 mit dem nicht-invertierenden Eingangsanschluss 15 verbunden, um eine unerwünschte hochfrequente Schwingungsneigung zu unterdrücken.

Die Leistungsverstärkungsstufe 22 kann nur dann sinnvoll nachgeregelt werden, wenn die von den Messsonden (vorliegend von den Messschleifen 6 und der Hallsonde 7) gelieferten Werte sowohl hinreichend genau als auch hinreichend schnell vorliegen. Der Grenzwert für "hinreichend genau und schnell" hängt dabei von dem jeweiligen Einsatzzweck ab. So erfordert beispielsweise ein therapeutischer Einsatz von Ionenstrahlen zur Tumortherapie ganz allgemein relativ scharfe Grenzwerte. Dabei sind die Grenzwerte für eine Tumortherapie im Rasterscanverfahren nochmals strenger als bei der Bestrahlung mit formgebenden Masken.

Nach dem derzeitigen Kenntnisstand genügt eine relative Stabilität der Magnetfelder von $10^{-4}$ (sowohl für Dipolfelder, als auch für Quadrupolfelder) für die Rasterscanbestrahlung von Tumoren sicher aus. Diese relative Bedingung muss jedoch für sämtliche verwendeten Strahlenergien erfüllt werden. Magnetfelder mit einer Stärke von 10% des maximalen Designwertes müssen also relativ gesehen auch auf $10^{-4}$ stabil sein, was einer Genauigkeit von $10^{-5}$ in Relation zum maximalen Designwert entspricht. Wird diese Genauigkeit nicht erreicht, so bringt die Nachregelung keine ausreichende Verbesserung gegenüber dem Ist-Zustand. Ganz im Gegenteil ist es möglich, dass sich die Teilchenstrahlqualität sogar verschlechtert.

Hinsichtlich der Geschwindigkeit, mit der die Messwerte für das Magnetfeld vorliegen müssen, haben die bisherigen Experimente ergeben, dass die Messwerte innerhalb von 30 μs, besser innerhalb von 20 μs, vorzugsweise innerhalb von 10 μs vorliegen sollten.

Das Geschwindigkeitserfordernis bringt es im Übrigen auch mit sich, dass bestimmte Verfahren, die bei einer Labormessung problemlos verwendet werden können, für eine "on line"-Nachregulierung nicht verwendet werden können. Beispielsweise ist eine nachträgliche Korrektur einer Drift im "on line"-Betrieb nicht möglich.

Im Normalbetrieb ist der Wahlschalter 19 so eingestellt, dass das auf dem gemessenen Magnetfeld beruhende erste Ansteuersignal 14 an den nicht-invertierenden Eingang 15 des Operationsverstärkers 16 angelegt ist. Das erste Ansteuersignal 14 wird von einer ersten elektronischen Verarbeitungsschaltung 25 bereitgestellt, welche die von den Messschleifen 6 und der Hallsonde 7 gemessenen Werte auf die bereits oben beschriebene Weise verarbeitet.

Sollte es zu einer Störung bei der Erzeugung des ersten Ansteuersignals 14 kommen (beispielsweise, weil die elektronische Verarbeitungsschaltung eine Inkonsistenz der Messsignale der Messschleifen 6 und/oder der Hallsonde 7 detektiert), so kann die Anlage in einem Notbetrieb weiter genutzt werden. Dazu wird der Wahlschalter 19 umgeschaltet, sodass nunmehr das Ausgangssignal 20 des Signaladdierers 21 an den nicht-invertierenden Eingang 15 des Operationsverstärkers 16 angelegt wird. Das Ausgangssignal 20 des Signaladdierers 21 folgt in der Hauptsache dem Ausgangssignal 12 einer elektronischen Steuerschaltung 26. Basierend auf dem aktuellen Betriebszustand des Synchrotrons erstellt diese aufgrund eines theoretischen Modells und unter Zuhilfenahme eines vorab in einem Messlabor ermittelten Kennlinienfelds, das die Beziehung zwischen angelegter Stromstärke und erzeugtem Magnetfeld wiedergibt, das Ausgangssignal 12, das mit der an den bzw. die Magneten (Dipolmagneten 1 und/oder Quadrupolmagneten 10) anzulegenden Stromstärke korreliert.

Zusätzlich wird an den Signaladdierer 21 ein weiteres Signal 13 angelegt. Das Signal 13 wird von einem DCCT (direct current to current transformer) 27 erzeugt. Dieser nimmt den tatsächlich in der Erregerspule 23 gemessenen Wert der Stromstärke 24 und vergleicht diesen mit der Sollvorgabe 12 der elektronischen Steuerschaltung 26. Basierend auf diesen Vergleich zwischen Sollwert 12 und Istwert 24 des in der Erregerspule 23 fließenden Stroms wird der Leistungsverstärker 22 entsprechend nachgestellt.

Im Notbetrieb entspricht die Ansteuerung der bislang üblichen Ansteuerung. Das heißt, dass nach wie vor ein "Kamin" oder Trainingszyklen verwendet werden müssen, um die für therapeutische Zwecke ausreichende Genauigkeit zu erreichen. Dennoch wird über den Notbetrieb eine erhöhte Betriebssicherheit der Gesamtanlage bereitgestellt.

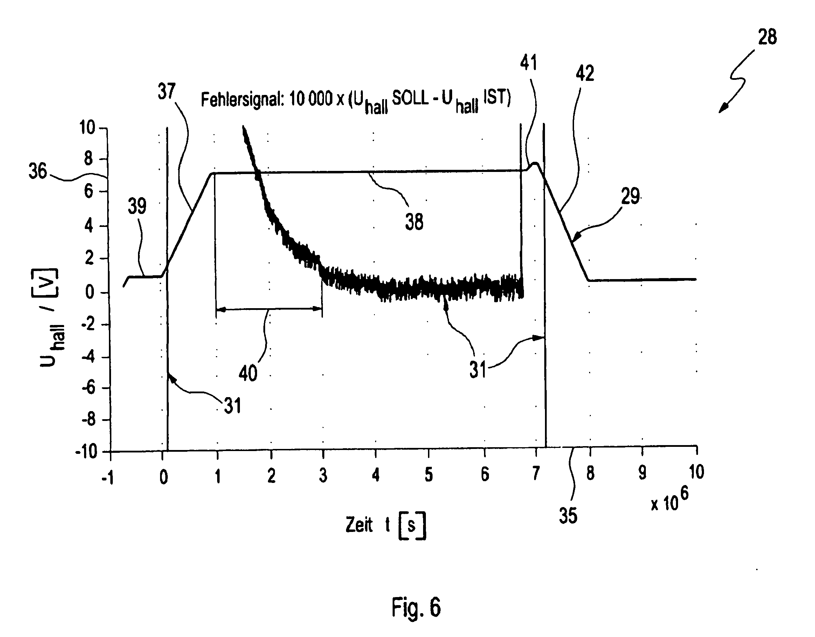

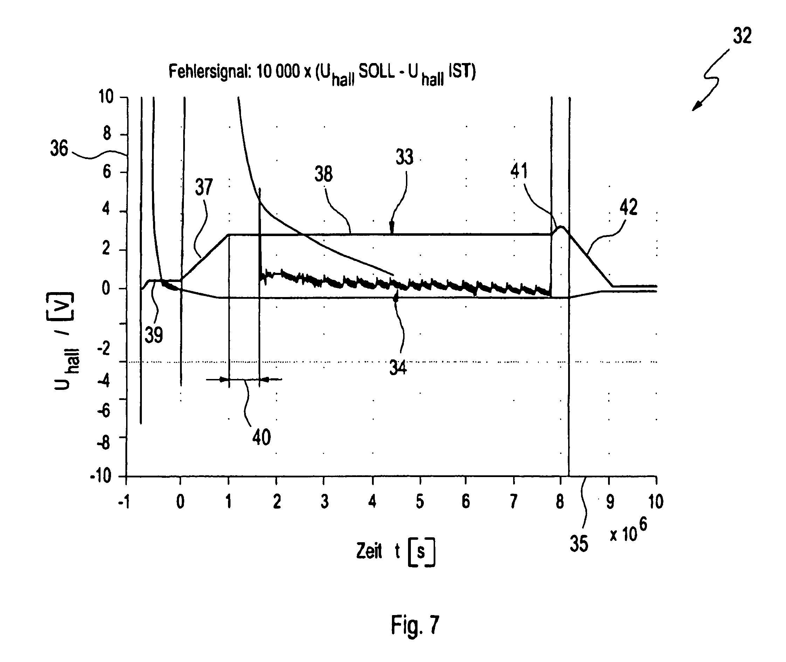

In Fig. 6 und Fig. 7 ist zum Vergleich jeweils ein Beschleunigungszyklus 28 im Notbetrieb (Fig. 6) sowie ein Beschleunigungszyklus 32 im Normalbetrieb (Fig. 7) dargestellt. Auf der Abszisse 35 ist jeweils die Zeit in Sekunden dargestellt. Auf der Ordinate 36 ist einerseits das Sollfeld 29, 33 des jeweiligen Magneten (in beliebigen Einheiten) dargestellt. Darüber hinaus ist entlang der Ordinate 36 die Differenz 31, 34 zwischen Sollfeld und Istfeld dargestellt, wobei die Differenz zur besseren Erkennbarkeit um den Faktor $10^4$ verstärkt ist.

Der Beschleunigungszyklus 28, 32 beginnt jeweils mit der Injektionsphase 39, zu der ein vorbeschleunigter Teilchenstrahl (beispielsweise ein Kohlenstoffionenstrahl) in das Synchrotron 75 injiziert wird.

Anschließend folgt die Beschleunigungsphase 37, während der die Teilchen beschleunigt werden. Während der Beschleunigungsphase 37 muss das von den Magneten 1, 10 erzeugte Magnetfeld mit zunehmender Teilchenenergie zunehmen. Wie aus den Fig. 6 und 7 zu erkennen ist, ist zu dieser Zeit auch die Differenz 31, 34 zwischen Sollwert und Istwert besonders hoch. Dies ist jedoch unproblematisch, da ein Synchrotron 75 zu diesem Zeitpunkt unter Betriebsbedingungen gefahren wird, die eine hohe Eigenstabilität des Teilchenstrahls 74 sicherstellen.

Nach dem Ende der Beschleunigungsphase 37 beginnt die Plateauphase 38, während der die Extraktion der Teilchen aus dem Synchrotron 75 erfolgen soll. Um die Teilchen extrahieren zu können ist es jedoch erforderlich, das Synchrotron 75 zunächst in einen Betriebszustand zu bringen, bei der der Teilchenstrahl 74 nur eine geringe Eigenstabilität aufweist. Dies setzt aber voraus, dass die Differenz 31, 34 zwischen Sollwert und Istwert des Magnetfelds sehr gering ist. Ansonsten kann es zu einem Strahlverlust kommen. Aber auch selbst wenn es zu keinem Strahlverlust käme, wäre eine größere Differenz 31, 34 zwischen Sollwert und Istwert des Magnetfelds problematisch, da dies unter anderem zur Folge hätte, dass die Teilchen des Teilchenstrahls 74 nicht gleichmäßig (weitgehend konstante Anzahl extrahierter Teilchen pro Zeiteinheit), sondern mit einem ausgeprägten Peak extrahiert würden. Dies ist jedoch speziell für medizinische Anwendungen unerwünscht, da sich die Therapieergebnisse verschlechtern. Auch sonstige Eigenschaften des Teilchenstrahls können sich verschlechtern, was ebenfalls unerwünscht ist.

Aus diesem Grund ist es im Notbetrieb erforderlich, zunächst noch eine relativ lange Stabilisierungsphase 40 abzuwarten, bis die die Differenz 31, 34 zwischen Sollwert und Istwert des Magnetfelds hinreichend klein ist. Die Zeitspanne der Stabilisierungsphase 40 muss so groß sein, dass die dynamischen Störungseffekte, die im Wesentlichen durch Wirbelströme hervorgerufen werden, in ausreichendem Maße abklingen können. In der Regel beträgt die Zeitdauer der Stabilisierungsphase 40 ein Mehrfaches der Zeitkonstante des Jochmaterials. Vorliegend muss eine Zeitdauer von etwa 2 Sekunden abgewartet werden (bei einer Gesamtzyklusdauer von etwas 10 Sekunden). Erst anschließend kann mit der Extraktion des Teilchenstrahls begonnen werden. Es ist unmittelbar ersichtlich, dass die Notwendigkeit mehrere Sekunden abwarten zu müssen, die Effizienz der Anlage verschlechtert.

Demgegenüber ist die Stabilisierungsphase 40 im Normalbetrieb 32 (Fig. 7) deutlich verkürzt. Es kann somit deutlich früher mit der Extraktion des Teilchenstrahls 74 begonnen werden.

Im Anschluss an die Plateauphase 38 wird bei dem in Fig. 6 und 7 dargestellten Ausführungsbeispielen sowohl im Notbetrieb 28, als auch im Normalbetrieb 32 ein sogenannter "Kamin" 41 gefahren. Das heißt, das Magnetfeld wird nochmals erhöht, bis sich das weichmagnetische Material des Jochs 4 der Magneten 1, 10 in einem Sättigungszustand befindet. Dies ist - wie bereits erwähnt - notwendig, um für den nächsten Beschleunigungszyklus 28, 32 definierte Ausgangsbedingungen für das Jochmaterial 4 zu schaffen. Im Anschluss an den "Kamin" 41 wird das Magnetfeld auf das Niveau der Injektionsphase 39 zurückgefahren 42.

Es wird darauf hingewiesen, dass die in Fig. 6 und 7 dargestellten Beschleunigungszyklen 28, 32 in einer Prototypanordnung gemessen wurden. Aktuelle Versuchsergebnisse deuten darauf hin, dass es im Normalbetrieb 32 (Fig. 7) voraussichtlich möglich ist, den "Kamin" 41 vollständig oder zumindest teilweise weglassen zu können. Es ist unmittelbar ersichtlich, dass dadurch erneut Energie und Totzeit der Anlage eingespart werden kann. Dies ist insbesondere dann der Fall, wenn niedrige Energien gefahren werden. Dann ist zwar die Beschleunigungsphase 37 deutlich kürzer; der Zeitbedarf für einen "Kamin" verlängert sich jedoch entsprechend.

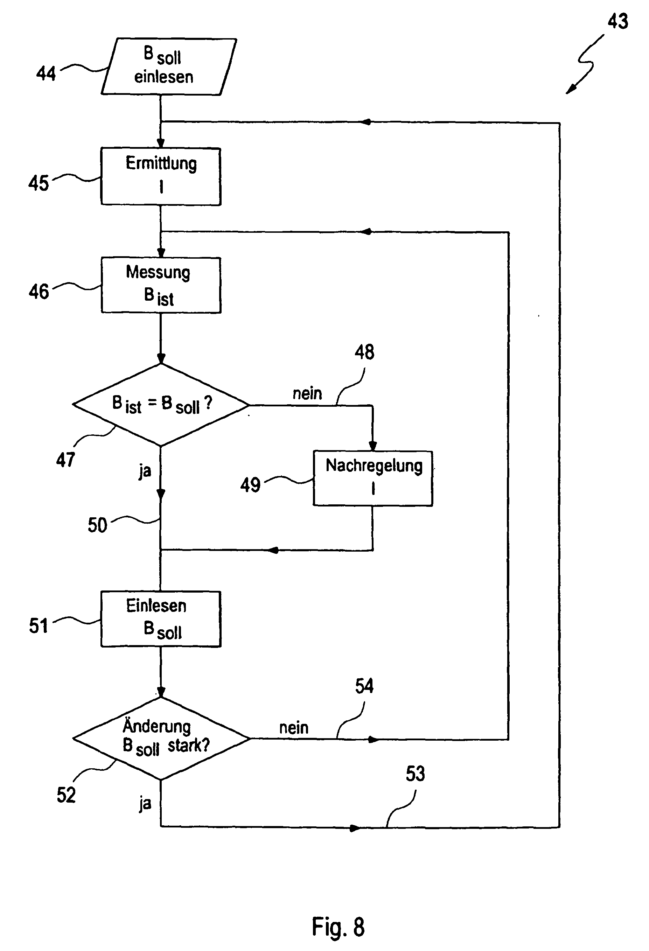

In Fig. 8 ist ein Flussdiagramm 43 für ein mögliches Verfahren zur Magnetfeldregelung eines Beschleunigermagneten (z.B. eines Dipolmagneten 1 oder eines Quadrupolmagneten 10) für ein zur Tumorbehandlung verwendetes Synchrotron 75 dargestellt.

Zu Beginn des Verfahrens wird in einem ersten Schritt 44 ein Anfangs-Sollwert $B_{soll}$ für die vom Magneten 1, 10 zu erzeugende Magnetfeldstärke eingelesen.

Basierend auf dem Sollwert der Magnetfeldstärke $B_{soll}$ werden in einem zweiten Schritt 45 Anfangsparameter für den Magneten 1, 10, insbesondere eine zunächst einzustellende Stromstärke I ermittelt. Dies kann beispielsweise unter Verwendung einer vorab erstellten "Look-up"-Tabelle erfolgen. Die so ermittelten Werte werden an den Magneten 1, 10 angelegt. Vorzugsweise wird eine gewisse Zeitspanne gewartet, bis sich der Magnet 1, 10 stabilisiert hat. Die abzuwartende Zeitspanne kann beispielsweise ebenfalls in der "Look-up"-Tabelle hinterlegt sein. Ebenfalls ist es möglich, dass der Stromstärkeregler (DCCT-Regler) ein Signal ausgibt, dass er die Soll-Stromstärke I erreicht hat.

Anschließend wird in einem weiteren Schritt 46 das mit Hilfe der Magnetfeldsensoren ermittelte Magnet-

feld $B_{ist}$ gemessen. Der so gewonnene Messwert $B_{ist}$ wird anschließend mit dem Sollwert $B_{soll}$ verglichen. Weichen die Werte $B_{soll}$ und $B_{ist}$ voneinander ab, so verzweigt das Verfahren in einen ersten Zweig 48. In diesem Zweig 48 wird basierend auf der Differenz zwischen $B_{soll}$ und $B_{ist}$ die Magnetstromstärke I nachgeregelt 49. Stimmen die Werte von $B_{soll}$ und $B_{ist}$ dagegen überein, so wird der Nachregelungsschritt 49 übersprungen und der Verfahrensablauf 50 geht direkt zum nächsten Schritt 51 über.

Im nun folgenden Schritt 51 wird der nunmehr aktuelle Magnetfeldstärkesollwert $B_{soll}$ eingelesen. Im Anschluss daran wird überprüft 52, ob der neue Magnetfeldstärkesollwert $B_{soll}$ stark 53 oder nur schwach (falls überhaupt) 54 vom vorherigen Magnetfeldstärkesollwert $B_{soll}$ abweicht. Dementsprechend verzweigt der Verfahrensablauf. Das Kriterium "starke Abweichung" bzw. "schwache Abweichung" kann beispielsweise auf einer absoluten Änderung des Magnetfeldstärkesollwerts $B_{soll}$ oder auf einer relativen Änderung des Magnetfeldstärkesollwerts $B_{soll}$ basieren.

Ändert sich der Magnetfeldstärkesollwert $B_{soll}$ nicht oder nur wenig verzweigt der Verfahrensablauf 54 zurück zum Anfang der Nachregelschleife 46, 47, 49, also zum Einlesen 46 des Magnetfeldstärkeistwerts $B_{ist}$.

Ändert sich der Magnetfeldstärkesollwert $B_{soll}$ dagegen stark, so springt der Verfahrensablauf 53 weiter zurück. Es wird nun zunächst erneut ein Anfangsmagnetstromwert I ermittelt 45. Erst anschließend wird wieder die Nachregelschleife 46, 47, 49 durchgeführt.

Selbstverständlich ist es auch möglich, beim Verfahrensablauf 43 ein Abbruchkriterium vorzusehen, beispielsweise wenn die Anlage, auf der das Verfahren durchgeführt wird, ausgeschaltet werden soll. Beispielsweise kann beim Vorliegen eines Abbruchsignals die Magnetstromstärke auf Null verfahren werden.

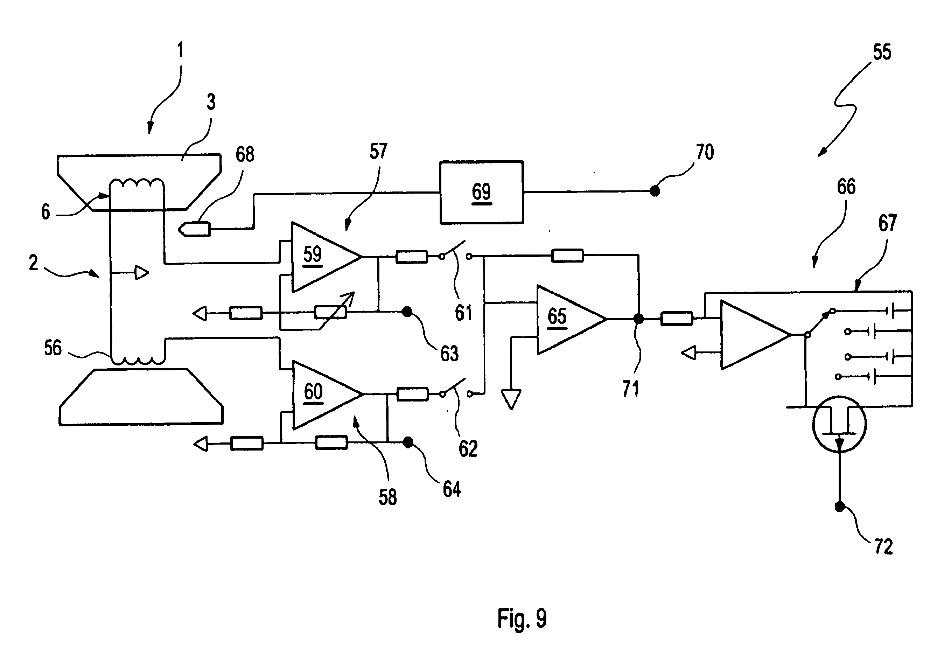

In Fig. 9 ist ein schematischer Schaltplan 55 einer Schaltung zur Kalibrierung einer Messschleife ("pick-up coil") 6 dargestellt. Die Messschleife 6 ist um den Polschuh 3 des in Fig. 9 nur schematisch dargestellten Dipolmagneten 1 herum angeordnet. Zusätzlich ist in der Mittelausnehmung 2 des Dipolmagneten 1 eine Referenzspule 56 angeordnet, die insbesondere in einem Bereich angeordnet werden kann, in dem in fertig aufgebauten Zustand der Anlage 73 der Teilchenstrahl 74 geführt wird (oder gegebenenfalls ein anderer Gegenstand angeordnet wird). Die von den Messschleifen 6, 56 aufgenommenen Messwerte werden zunächst zwei Verstärkereinheiten 57, 58 zugeführt, wobei jeweils eine Verstärkereinheit 57, 58 einer Messschleife 6, 56 zugeordnet sind. Die Verstärkereinheiten 57, 58 weisen neben einem Operationsverstärker 59, 60 zusätzliche Bauelemente (beispielsweise Widerstände) auf, mit denen die Verstärkereinheiten 57, 58 justiert werden können. Zusätzlich ist im Zusammenhang mit den Verstärkereinheiten 57, 58 jeweils ein Signalabgriffspunkt 63, 64 vorgesehen.

Den Verstärkereinheiten 57, 58 nachgeschaltet ist eine Nachverstärkerstufe 65. Über Schalter 61, 62 können die Ausgangssignale 63, 64 der Verstärkereinheiten 57, 58 selektiv der Nachverstärkerstufe 65 zugeführt werden. Das Ausgangssignal 71 der Nachverstärkerstufe 65 wird wiederum einer Integratorstufe 66 zugeführt, in der das Signal 71 aufintegriert wird. Der Integralwert wird als Ausgangssignal 72 von der Integratorstufe 66 ausgegeben. Die Integratorstufe 66 ist dabei über eine Einstelleinrichtung 67 auf unterschiedliche Verstärkungsstufen einstellbar. Ferner kann das Ausgangssignal 72 der Integratorstufe 66 mittels der Einstelleinrichtung 67 genullt werden.

Weiterhin ist in der Mittelausnehmung 2 des Dipolmagneten 1 eine Hallsonde 68 angeordnet, deren Ausgangssignal über eine Sondenelektronik 69 aufgearbeitet wird, so dass am Ausgang 70 der Sondenelektronik 69 ein Magnetfeldstärkewert 70 ausgegeben wird.

Bei geschlossenem Referenzspulenschalter 62 (Messschleifenschalter 61 offen) kann der Ausgabewert 70 der Hallsonde und der Ausgabewert 72 der Referenzspule 56 miteinander verglichen werden. Dabei kann die Verstärkereinheit 58 der Referenzspule 56 geeignet kalibriert werden. Bei geschlossenem Messschleifenschalter 61 (Referenzspulenschalter 62 offen) kann in gleicher Weise die Verstärkereinheit 59 der Messschleife 6 kalibriert werden.

Werden sowohl der Messschleifenschalter 61, als auch der Referenzspulenschalter 62 geschlossen, so wird (unterschiedliches Vorzeichen beachten!) die Differenz der Ausgabesignale 63, 64 von Messschleife 6 und Referenzspule 56 an die Nachverstärkereinheit 65 ausgegeben und von dieser verstärkt. Nunmehr kann der Unterschied zwischen beiden Messwerten 63, 64 quantitativ und qualitativ genau untersucht werden. Versuche haben dabei ergeben, dass die Korrelation der beiden Ausgabesignale 63, 64 von Messschleife 6 und Referenzspule 56 sehr gut sind. Insbesondere ist das Signal der Messschleife 6 ausreichend genau, um zur Nachregelung des Spulenstroms I der elektrischen Spulen 5 der Magneten 1, 10 geeignet zu sein.

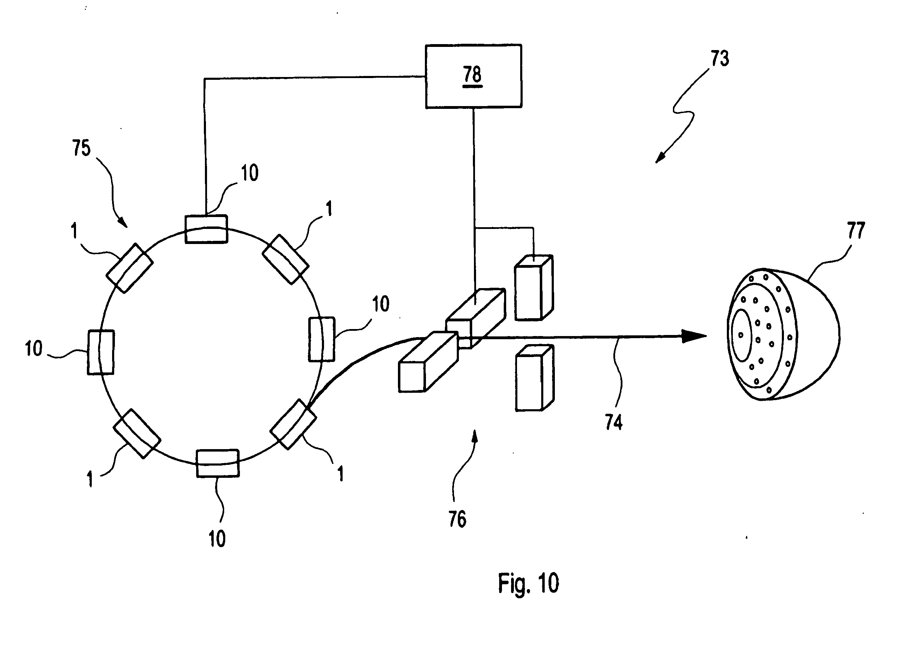

Fig. 10 skizziert einen Therapieanlage 73 zur Tumortherapie mit Hilfe eines Teilchenstrahls 74. Der Teilchenstrahl 74 wird von einem Synchrotron 75 erzeugt, das vorliegend nur schematisch skizziert ist. Das Synchrotron 75 weist eine Mehrzahl an Dipolmagneten 1 sowie an Quadrupolmagneten 10 auf. Der aus dem Synchrotron 75 extrahierte Teilchenstrahl 74 wird über ein Ablenkmagnetpaar 76 in zwei zueinander senkrecht stehenden Ebenen (x-Richtung, γ-Richtung) abgelenkt und einem Bestrahlungsgebiet 77 zugeführt. Bei dem Bestrahlungsgebiet 77 kann es sich insbesondere um einen zu bestrahlenden Tumor eines Patienten handeln. Während die Positionierung des Teilchenstrahls 74 auf das Bestrahlungsgebiet 77 in x-Richtung und γ-Richtung durch das Ablenkmagnetpaar 76 erfolgt, erfolgt die Positionierung in z-Richtung durch die Energie des Teilchenstrahls 74. Dadurch wird der Bragg-Peak des Teilchenstrahls 74 so gelegt, dass der hauptsächliche Energieverlust im Bestrahlungsgebiet 77 erfolgt. Der Behandlungsvorgang erfolgt, indem das Bestrahlungsgebiet 77 rasterartig abgefahren wird. Dazu muss die Strahlenergie des Teilchenstrahls 74 (und damit die Ansteuerung des Synchrotrons 75), als auch die Ablenkung des Teilchenstrahls 74 im Ablenkmagnetpaar 76 entsprechend variiert werden. Dies wird von einem Steuercomputer 78 übernommen, der mit einem entsprechenden Behandlungsplan gefüttert wurde.

Werden die Magnete 1, 10 des Synchrotrons 75 gemäß der vorgeschlagenen Bauweise ausgebildet und/oder gemäß des vorgeschlagenen Verfahrens angesteuert, so kann die Effektivität des Behandlungsvorgangs deutlich erhöht werden. Insbesondere kann die Behandlungsdauer verkürzt werden, was neben der für den Patienten vorteilhaften schnelleren Behandlung insbesondere auch eine größere Anzahl an Behandlungen ermöglicht.

**Patentansprüche**

1. Magnetfelderzeugungsvorrichtung (1, 10) mit wenigstens einem Magnetgap (2) zur Aufnahme von Gegenständen, auf die das Magnetfeld (29, 33), das von wenigstens einem Magnetfelderzeugungsmittel (5, 23) der Magnetfelderzeugungsvorrichtung (1, 10) erzeugt wird, einwirkt, wobei die Magnetfelderzeugungsvorrichtung (1, 10) als Magnetfelderzeugungsvorrichtung (1, 10) zur Verwendung in einem Synchrotron (75) ausgebildet ist, aufweisend wenigstens ein Magnetfeldmessmittel (6, 7), welches in einem außerhalb des Magnetgaps (2) befindlichen Messmittelaufnahmebereich angeordnet ist, **dadurch gekennzeichnet, dass** die Magnetfelderzeugungsvorrichtung so ausgebildet ist, dass zumindest ein Messsignal des wenigstens einen Magnetfeldmessmittels (6, 7) als Eingangssignal zur Ansteuerung (11) des wenigstens einen, das Magnetfeld (29, 33) der Magnetfelderzeugungsvorrichtung (1, 10) erzeugenden Magnetfelderzeugungsmittels (5, 23) verwendet wird.

2. Magnetfelderzeugungsvorrichtung (1, 10) nach Anspruch 1, **dadurch gekennzeichnet, dass** die Magnetfelderzeugungsvorrichtung (5, 23) zumindest zeitweise ein sich zeitlich veränderndes Magnetfeld (29, 33) erzeugt.

3. Magnetfelderzeugungsvorrichtung (1, 10) nach Anspruch 1 oder 2, **dadurch gekennzeichnet, dass** die Magnetfelderzeugungsvorrichtung (5, 23) zumindest eine Dipolmagnetfelderzeugungseinrichtung (1) und/oder zumindest eine Quadrupolmagnetfelderzeugungseinrichtung (10) aufweist.

4. Magnetfelderzeugungsvorrichtung (1, 10) nach einem der vorangehenden Ansprüche, **dadurch gekennzeichnet, dass** zumindest ein Magnetfeldmessmittel (6, 7) als Induktionsmessmittel (6), insbesondere als Leiterschleife und/oder Spule ausgebildet ist.

5. Magnetfelderzeugungsvorrichtung (1, 10) nach einem der vorangehenden Ansprüche, insbesondere nach Anspruch 4, **gekennzeichnet, durch** wenigstens eine Integratoreinrichtung (25).

6. Magnetfelderzeugungsvorrichtung (1, 10) nach einem der vorangehenden Ansprüche, insbesondere nach Anspruch 4 oder 5, **dadurch gekennzeichnet, dass** zumindest ein Magnetfeldmessmittel (6, 7) im Bereich zumindest eines Polschuhs (3) und/oder zumindest eines Jochs (4) angeordnet ist.

7. Magnetfelderzeugungsvorrichtung (1, 10) nach einem der vorangehenden Ansprüche, **dadurch gekennzeichnet, dass** zumindest ein Magnetfeldmessmittel (6, 7) als direktes Magnetfeldmessmittel (7), insbesondere als NMR-Sonde, als magnetoresistives Messelement und/oder als Hallsonde ausgebildet ist.

8. Magnetfelderzeugungsvorrichtung (1, 10) nach einem der vorangehenden Ansprüche, insbesondere nach einem der Ansprüche 5 bis 7, **dadurch gekennzeichnet, dass** zumindest ein Magnetfeldmessmittel (7) als Triggereinrichtung verwendet wird.

9. Anlage (73) mit wenigstens einer Magnetfelderzeugungsvorrichtung (1, 10, 76), **dadurch gekennzeichnet, dass** wenigstens eine der Magnetfelderzeugungsvorrichtungen (1, 10, 76) gemäß einem der Ansprüche 1 bis 8 ausgebildet ist.

10. Anlage (73) nach Anspruch 9, **dadurch gekennzeichnet, dass** die Anlage (73) zumindest teilweise als medizinische Therapieeinrichtung, als medizinische Diagnoseeinrichtung, als Computertomograph, als Kernspintomograph und/oder als Teilchenbeschleuniger, insbesondere als Linearbeschleuniger und/oder als Synchroton (75) ausgebildet ist.

11. Anlage (73) nach Anspruch 9 oder 10, **gekennzeichnet durch** ein Synchrotron (75), wobei das Synchrotron (75) zumindest eine Magnetfelderzeugungsvorrichtung (1, 10, 76) aufweist, welche als Dipolmagnetfelderzeugungsvorrichtung (1, 76) und/oder als Quadrupolmagnetfelderzeugungsvorrichtung (10) ausgebildet ist, wobei die zumindest eine Magnetfelderzeugungsvorrichtung (1, 10, 76) zumindest ein Magnetfeldmessmittel (6, 7) umfasst, das in einem Magnetgap (2) der zumindest einen Magnetfelderzeugungsvorrichtung (1, 10, 76) angeordnet ist, wobei der Messwert des zumindest einen Magnetfeldmessmittels (6, 7) zur Ansteuerung und/oder Nachregelung zumindest einer Magnetfelderzeugungsvorrichtung (1, 10, 76), insbesondere zur Ansteuerung der Magnetfelderzeugungsvorrichtung (1, 10, 76), in dem das zumindest eine Magnetfeldmessmittel (6, 7) angeordnet ist, verwendet wird.

12. Verfahren zum Betrieb einer Magnetfelderzeugungsvorrichtung (1, 10, 76) und/oder einer Anlage (73) mit wenigstens einer Magnetfelderzeugungsvorrichtung (1, 10, 76), wobei die Magnetfelderzeugungsvorrichtung (1, 10, 76) als Magnetfelderzeugungsvorrichtung (1, 10, 76) zur Verwendung in einem Synchrotron (73) ausgebildet ist, insbesondere Verfahren zum Betrieb einer Magnetfelderzeugungsvorrichtung (1, 10, 76) nach einem der Ansprüche 1 bis 8 und/oder einer Anlage (73) nach Anspruch 9 bis 11, **dadurch gekennzeichnet, dass** die Magnetfelderzeugungsvorrichtung (1, 10, 76) derart betrieben wird, dass das Magnetfeld mit Hilfe eines Magnetfeldmessmittels (6, 7) gemessenen wird und der gemessene Wert des Magnetfelds zur Ansteuerung (11) des zumindest einen Magnetfelderzeugungsmittels (5, 23), welches das Magnetfeld der Magnetfelderzeugungsvorrichtung (1, 10, 76) erzeugt, verwendet wird.

13. Verfahren nach Anspruch 12, **dadurch gekennzeichnet, dass** der gemessene Wert des Magnetfelds zur Ansteuerung einer Stromversorgungsvorrichtung (22) des Magnetfelderzeugungsmittels (5, 23) verwendet wird.

14. Verfahren nach Anspruch 12 oder 13, **dadurch gekennzeichnet, dass** die Ansteuerung zumindest eines Magnetfelderzeugungsmittels (1, 10) zumindest zeitweise und/oder zumindest bereichsweise und/ oder zumindest teilweise in Abhängigkeit von dem von dem Magnetfeldmessmittel (6, 7) gemessenen Magnetfeld erfolgt.

15. Verfahren nach einem der Ansprüche 12 bis 14, **dadurch gekennzeichnet, dass** die Ansteuerung zumindest zeitweise und/oder zumindest bereichsweise und/oder zumindest teilweise gemäß einem vorab ermittelten Ansteuerungsmodell (26) erfolgt, welches zumindest zeitweise und/oder zumindest bereichsweise und/oder zumindest teilweise in Abhängigkeit von dem von dem Magnetfeldmessmittel (6, 7) gemessenen Magnetfeld nachgeführt (19) wird.

**Claims**

1. Magnetic field generating device (1, 10) having at least one magnetic gap (2) for accommodating objects on which acts the magnetic field (29, 33) which is generated by at least one magnetic field generating means (5, 23) of the magnetic field generating device (1, 10), where the magnetic field generating device (1, 10) is designed as a magnetic field generating device (1, 10) for use in a synchrotron (75) and comprises at least one magnetic field measuring means (6, 7) arranged in a measurement means receiving area located outside the magnetic gap (2), **characterized in that** the magnetic field generating device is designed such that at least one measurement signal of the at least one magnetic field measuring means (6, 7) is used as the input signal for control (11) of the at least one magnetic field generating means (5, 23) creating the magnetic field (29, 33) of the magnetic field generating device (1, 10).

2. Magnetic field generating device (1, 10) according to claim 1, **characterized in that** the magnetic field generating device (5, 23) generates at least at times a magnetic field (29, 33) varying over time.

3. Magnetic field generating device (1, 10) according to claim 1 or 2, **characterized in that** the magnetic field generating device (5, 23) comprises at least one dipole magnetic field generating device (1) and/or at least one quadrupole magnetic field generating device (10).

4. Magnetic field generating device (1, 10) according to one of the preceding claims, **characterized in that** at least one magnetic field measuring means (6, 7) is designed as an inductive measuring means (6), in particular as a conductive loop and/or as a coil.

5. Magnetic field generating device (1, 10) according to one of the preceding claims, in particular according to claim 4, **characterized by** at least one integrator unit (25).

6. Magnetic field generating device (1, 10) according to one of the preceding claims, in particular according to claim 4 or 5, **characterized in that** at least one magnetic field measuring means (6, 7) is arranged in the region of at least one pole piece (3) and/or of at least one yoke (4).

7. Magnetic field generating device (1, 10) according to one of the preceding claims, **characterized in that** at least one magnetic field measuring means (6, 7) is designed as a direct magnetic field measuring means (7), in particular as an NMR probe, as a magneto-resistive measuring element and/or as a Hall probe.

8. Magnetic field generating device (1, 10) according to one of the preceding claims, in particular according to one of claims 5 to 7, **characterized in that** at least one magnetic field measuring means (7) is used as a trigger unit.

9. System (73) having at least one magnetic field generating device (1, 10, 76), **characterized in that** at least one of the magnetic field generating devices (1, 10, 76) is designed according to one of claims 1 to 8.

10. System (73) according to claim 9, **characterized in that** the system (73) is designed at least in part as medical therapy unit, as medical diagnostics unit, as a computer tomograph, as a nuclear spin tomograph and/or as a particle accelerator, in particular as a linear accelerator and/or as a synchrotron (75).

11. System (73) according to claim 9 or 10, **characterized by** a synchrotron (75), where the synchrotron (75) comprises at least one magnetic field generating device (1, 10, 76) designed as a dipole magnetic field generating device (1, 76) and/or as a quadrupole magnetic field generating device (10), where the at least one magnetic field generating device (1, 10, 76) comprises at least one magnetic field measuring means (6, 7) arranged in a magnetic gap (2) of the at least one magnetic field generating device (1, 10, 76), where the measurement value from the at least one magnetic field measuring means (6, 7) is used for control and/or readjustment of at least one magnetic field generating device (1, 10, 76), in particular for control of the magnetic field generating device (1, 10, 76) in which the at least one magnetic field measuring means (6, 7) is arranged.

12. Method for operation of a magnetic field generating device (1, 10, 76) and or of a system (73) having at least one magnetic field generating device (1, 10, 76), where the magnetic field generating device (1, 10, 76) is designed as a magnetic field generating device (1, 10, 76) for use in a synchrotron (73), in particular method for operation of a magnetic field generating device (1, 10, 76) according to one of claims 1 to 8 and/or of a system (73) according to claims 9 to 11, **characterized in that** the magnetic field generating device (1, 10, 76) is operated such that the magnetic field is measured with the aid of a magnetic field measuring means (6, 7) and the measured value of the magnetic field is used for control (11) of the at least one magnetic field generating means (5, 23) which generates the magnetic field of the magnetic field generating device (1, 10, 76).

13. Method according to claim 12, **characterized in that** the measured value of the magnetic field is used for control of a power supply device (22) of the magnetic field generating means (5, 23).

14. Method according to claim 12 or 13, **characterized in that** the control of at least one magnetic field generating means (1, 10) is carried out at least at times and/or at least in some regions and/or at least partially depending on the magnetic field measured by the magnetic field measuring means (6, 7).

15. Method according to one of claims 12 to 14, **characterized in that** the control is carried out at least at times and/or at least in some regions and/or at least partially according to a previously determined control model (26) which is updated (19) at least at times and/or at least in some regions and/or at least partially depending on the magnetic field (19) measured by the magnetic field measuring means (6, 7).

**Revendications**

1. Dispositif générateur de champ magnétique (1, 10) avec au moins un gap magnétique (2) servant à la réception d'objets, le champ magnétique (29, 33) agissant sur ledit objet, ledit champ étant généré par au moins un moyen de génération de champ magnétique (5, 23) du dispositif générateur de champ magnétique (1, 10), sachant que le dispositif générateur de champ magnétique (1, 10) est conçu sous la forme d'un dispositif générateur de champ magnétique (1, 10) pour l'utilisation dans un synchrotron (75), présentant au moins un moyen de mesure du champ magnétique (6, 7), lequel est disposé dans une zone de logement du moyen de mesure située en dehors du gap magnétique (2),
**caractérisé en ce**
**que** le dispositif générateur de champ magnétique est conçu de sorte qu'au moins un signal de mesure de l'au moins un moyen de mesure du champ magnétique (6, 7) est utilisé comme signal d'entrée pour la commande (11) d'au moins un moyen de génération de champ magnétique (5, 23) générant le champ magnétique (29, 33) du dispositif générateur de champ magnétique (1, 10).

2. Dispositif générateur de champ magnétique (1, 10) selon la revendication 1,
**caractérisé en ce**
**que** le dispositif générateur de champ magnétique (5, 23) génère au moins temporairement un champ magnétique (29, 33) se modifiant dans le temps.

3. Dispositif générateur de champ magnétique (1, 10) selon la revendication 1 ou 2,
**caractérisé en ce**
**que** le dispositif générateur de champ magnétique (5, 23) présente au moins un dispositif générateur de champ magnétique dipolaire (1) et/ou au moins un dispositif générateur de champ magnétique quadripolaire (10).

4. Dispositif générateur de champ magnétique (1, 10) selon l'une des revendications précédentes, **caractérisé en ce**

**qu'**au moins un moyen de mesure du champ magnétique (6, 7) est conçu sous forme de moyen de mesure à induction (6), notamment sous forme de boucle conductrice et/ou de bobine.

5. Dispositif générateur de champ magnétique (1, 10) selon l'une des revendications précédentes, en particulier selon la revendication 4, **caractérisé par** au moins un dispositif d'intégration (25).

6. Dispositif générateur de champ magnétique (1, 10) selon l'une des revendications précédentes, en particulier selon la revendication 4 ou 5, **caractérisé en ce**

**qu'**au moins un moyen de mesure du champ magnétique (6, 7) est disposé dans le secteur d'au moins une masse polaire (3) et/ou d'au moins une culasse (4).

7. Dispositif générateur de champ magnétique (1, 10) selon l'une des revendications précédentes, **caractérisé en ce**

**qu'**au moins un moyen de mesure du champ magnétique (6, 7) est conçu sous la forme d'un moyen de mesure direct du champ magnétique (7), notamment sous la forme d'une sonde RMN, d'un élément de mesure magnéto-résistant et/ou d'une sonde de Hall.

8. Dispositif générateur de champ magnétique (1, 10) selon l'une des revendications précédentes, en particulier selon l'une des revendications 5 à 7, **caractérisé en ce**

**qu'**au moins un moyen de mesure du champ magnétique (7) est utilisé comme dispositif de déclenchement.

9. Installation (73) avec au moins un dispositif générateur de champ magnétique (1, 10, 76), **caractérisée en ce**

**qu'**au moins un des dispositifs générateurs de champ magnétique (1, 10, 76) est conçu conformément à l'une des revendications 1 à 8.

10. Installation (73) selon la revendication 9, **caractérisée en ce**

**que** l'installation (73) est conçue au moins partiellement sous forme de dispositif thérapeutique médical, de dispositif de diagnostic médical, de tomographe assisté par ordinateur, de tomographe à résonance magnétique nucléaire et/ou d'accélérateur de particules, notamment sous forme d'accélérateur linéaire et/ou de synchrotron (75).

11. Installation (73) selon la revendication 9 ou 10, **caractérisée par** un synchrotron (75), sachant que le synchrotron (75) présente au moins un dispositif générateur de champ magnétique (1, 10, 76), lequel est conçu sous forme de dispositif générateur de champ magnétique dipolaire (1, 76) et/ou de dispositif générateur de champ magnétique quadripolaire (10), sachant que l'au moins un dispositif générateur de champ magnétique (1, 10, 76) comprend au moins un moyen de mesure du champ magnétique (6, 7), lequel est disposé dans un gap magnétique (2) d'au moins un dispositif générateur de champ magnétique (1, 10, 76), sachant que la valeur de mesure d'au moins un moyen de mesure du champ magnétiques (6, 7) est utilisée pour la commande et/ou le réglage ultérieur d'au moins un dispositif générateur de champ magnétique (1, 10, 76), en particulier pour la commande du dispositif générateur de champ magnétique (1, 10, 76) dans lequel se trouve l'au moins un moyen de mesure du champ magnétique (6, 7).

12. Procédé de fonctionnement d'un dispositif générateur de champ magnétique (1, 10, 76) et/ou d'une installation (73) avec au moins un dispositif générateur de champ magnétique (1, 10, 76), sachant que le dispositif générateur de champ magnétique (1, 10, 76) est conçu sous forme d'un dispositif générateur de champ magnétique (1, 10, 76) pour l'utilisation dans un synchrotron (73), notamment procédé de fonctionnement d'un dispositif générateur de champ magnétique (1, 10, 76) selon l'une des revendications 1 à 8 et/ou d'une installation (73) selon la revendication 9 à 11, **caractérisé en ce**

**que** le dispositif générateur de champ magnétique (1, 10, 76) fonctionne de sorte que le champ magnétique est mesuré à l'aide d'un moyen de mesure du champ magnétiques (6, 7) et que la valeur mesurée du champ magnétique est utilisée pour la commande (11) de l'au moins un moyen de génération de champ magnétique (5, 23), lequel génère le champ magnétique du dispositif générateur de champ magnétique (1, 10, 76).

13. Procédé selon la revendication 12, **caractérisé en ce**

**que** la valeur mesurée du champ magnétique est utilisée pour la commande d'un dispositif d'alimentation électrique (22) du moyen de génération de champ magnétique (5, 23).

14. Procédé selon la revendication 12 ou 13, **caractérisé en ce**

**que** la commande d'au moins un moyen de génération de champ magnétique (1, 10) se fait au moins temporairement et/ou au moins par zones et/ou au moins partiellement en fonction du champ magnétique mesuré par le moyen de mesure du champ magnétique (6, 7).

**15.** Procédé selon l'une des revendications 12 à 14, **caractérisé en ce que** la commande se fait au moins temporairement et/ou au moins par zones et/ou au moins partiellement selon un modèle de commande (26) déterminé au préalable, lequel est asservi (19) au moins temporairement et/ou au moins par zones et/ou au moins partiellement en fonction du champ magnétique mesuré par le moyen de mesure du champ magnétique (6, 7).

Fig. 1

Fig. 2

Fig. 3

Fig. 4

Fig. 5

Fig. 6

Fehlersignal: 10 000 x (U$_{hall}$ SOLL - U$_{hall}$ IST)

Fig. 7

EP 2 274 634 B1

Fig. 8

Fig. 9

EP 2 274 634 B1

Fig. 10

EP 2 274 634 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4419061
- EP 1603142 A
- US 6635883 B